# EUROPEAN PATENT APPLICATION

(11) **EP 4 365 197 A1**
(43) Date of publication of application: **08.05.2024**
(21) Application number: 21948581.0
(22) Date of filing: 07.12.2021
(51) Int. Cl.: C07K 16/28, A61P 35/00, A61P 35/02, A61K 39/00

(54) **HUMANIZED ANTIBODY SPECIFIC TO CD47 AND PHARMACEUTICAL COMPOSITION COMPRISING SAME FOR PREVENTING OR TREATING CD47-RELATED DISEASE**

(30) Priority: 30.06.2021 KR 20210085561
(71) Applicant: Innobation Bio Co., Ltd., Seoul 03929 (KR)
(72) Inventor: KIM, Seung Ku, Yongin-si, Gyeonggi-do 16865 (KR); KIM, Ki Tae, Seoul 06148 (KR)
(74) Representative: J A Kemp LLP
(86) International application number: PCT/KR2021/018466
(87) International publication number: WO 2023/277281

(57) **Abstract**

The present invention relates to a humanized antibody specific for CD47 and a pharmaceutical composition for preventing or treating a CD47-related disease comprising the same, and more particularly, a humanized antibody specific for CD47, and a CD47-overexpressing cell comprising the humanized antibody It relates to a pharmaceutical composition for preventing or treating diseases mediated by

In the present invention, 16 kinds of humanized antibodies were prepared using an antibody (3A5 antibody) that binds to CD47, and it was confirmed that the humanized anti-CD47 antibody specifically binds to the CD47 antigen.

In addition, the humanized anti-CD47 antibody of the present invention not only blocks CD47-SIRPα binding, but also promotes phagocytosis by macrophages by binding to cells overexpressing CD47, and inhibits the growth of CD47-expressing tumors. Therefore, it can be applied to the prevention or treatment of diseases or tumors in which the immune response by overexpression of CD47 is suppressed.

## Description

### [Technical field]

The present invention relates to a humanized antibody specific for CD47 and a pharmaceutical composition for preventing or treating a CD47-related disease comprising the same, and more particularly, a humanized antibody specific for CD47, and a pharmaceutical composition for preventing or treating a CD47-related disease comprising the humanized antibody.

### [Background art]

CD47, also called integrin-binding protein (IAP), is a transmembrane glycoprotein widely expressed on the cell surface. It interacts with various ligands such as thrombospondin.

SIRPα is mainly expressed in bone marrow cells, including macrophages, granulocytes, myeloid dendritic cells (DCs), mast cells, hematopoietic stem cells (HSCs) and their precursors. SIRPα inhibits phagocytosis of host cells by macrophages, and ligation of SIRPα on macrophages by CD47 expressed on host target cells produces a SHP-1 mediated inhibitory signal, thereby negatively regulates to phagocytosing.

In the innate immune system, CD47 functions through binding to SIRPα expressed in myeloid cells, and the action of broad expression of CD47 under physiological conditions prevents healthy cells from being cleared by the innate immune system.

However, in recent years, CD47 and CD47-SIRPα signaling systems have received the most attention as potential drug targets in tumor therapy, as tumor cells can effectively evade immune surveillance by overexpression of CD47. Previous studies have shown that CD47 expression is upregulated and elevated in most human cancers (e.g., NHL, AML, breast cancer, colon cancer, glioblastoma, glioma, ovarian cancer, bladder cancer and prostate cancer). Expression levels of CD47 have been demonstrated to be associated with invasive disease and low survival rates.

Treatment of tumors with anti-CD47 antibodies involves a variety of mechanisms. First, the anti-CD47 antibody blocks the binding of CD47 on tumor cells to SIRPα on macrophages, allowing tumor cells to be phagocytosed. In addition, the anti-CD47 antibody can induce cytotoxicity of tumor cells involving NK cells, and can eliminate tumor cells by directly inducing apoptosis. Finally, anti- CD47 antibody can activate CD8+ T cells and induce an immune response of acquired T cells to further kill tumor cells.

For the treatment or diagnosis of such CD47 overexpressing tumor cells or CD47 overexpression-related diseases, CD47-specific antibodies are being developed, International Patent Publication No. WO2018-075857, International Patent Publication No. WO2017-121771 and International Publication Patent WO2013-119714 discloses various anti-CD47 antibodies.

As described above, monoclonal antibodies are mainly produced using mice for the production of antibodies for treatment. However, since non-human antibodies such as mouse-derived monoclonal antibodies are considered foreign antigens in the human body, they induce an immune response and have a limited therapeutic effect because of their short half-life.

In order to solve the above problem, a humanized antibody has been developed in which the rest of the antibody except for the antigen-binding CDR region is substituted with a human antibody. As a method of replacing a mouse antibody with a humanized antibody currently used, a human antibody gene most similar to the antibody to be replaced is selected, and only the CDR regions of a mouse antibody are replaced with human antibody CDR positions by a method called CDR grafting. These humanized antibodies have the advantage of reducing the immune response in the human body because most of the genes are humanized.

### [Disclosure]

### [Technical Problem]

In the present invention, an antibody (3A5) that bound to CD47 was selected to reduce the immune response in the human body, and a humanized anti-CD47 antibody was prepared using this. It was confirmed that the humanized anti-CD47 antibody prepared in the present invention specifically bound to the CD47 antigen, and effectively blocked CD47-SIRPα binding, as well as induced apoptosis of peripheral leukemia-derived T cells (Jurkat cells) and effectively inhibited tumor growth, and the present invention has been completed.

Accordingly, an object of the present invention is to provide a humanized antibody that specifically binds to CD47, a polynucleotide encoding the antibody, a vector expressing the antibody, and a recombinant cell transformed with the vector.

Another object of the present invention is to provide a pharmaceutical composition for preventing or treating a disease mediated by cells overexpressing CD47, including the humanized antibody specifically binding to CD47.

### [Technical solution]

In order to achieve the above object, the present invention relates to a humanized antibody or fragment thereof that specifically binds to CD47, comprising:
(1) a heavy chain variable region represented by the amino acid sequence of SEQ ID NO: 11 and a light chain variable region represented by the amino acid sequence of SEQ ID NO: 12;
(2) a heavy chain variable region represented by the amino acid sequence of SEQ ID NO: 17 and a light chain variable region represented by the amino acid sequence of SEQ ID NO: 18;
(3) a heavy chain variable region represented by the amino acid sequence of SEQ ID NO: 23 and a light chain variable region represented by the amino acid sequence of SEQ ID NO: 24;
(4) a heavy chain variable region represented by the amino acid sequence of SEQ ID NO: 29 and a light chain variable region represented by the amino acid sequence of SEQ ID NO: 30;
(5) a heavy chain variable region represented by the amino acid sequence of SEQ ID NO: 35 and a light chain variable region represented by the amino acid sequence of SEQ ID NO: 36;
(6) a heavy chain variable region represented by the amino acid sequence of SEQ ID NO: 41 and a light chain variable region represented by the amino acid sequence of SEQ ID NO: 42;
(7) a heavy chain variable region represented by the amino acid sequence of SEQ ID NO: 47 and a light chain variable region represented by the amino acid sequence of SEQ ID NO: 48;
(8) a heavy chain variable region represented by the amino acid sequence of SEQ ID NO: 53 and a light chain variable region represented by the amino acid sequence of SEQ ID NO: 54;
(9) a heavy chain variable region represented by the amino acid sequence of SEQ ID NO: 59 and a light chain variable region represented by the amino acid sequence of SEQ ID NO: 60;
(10) a heavy chain variable region represented by the amino acid sequence of SEQ ID NO: 65 and a light chain variable region represented by the amino acid sequence of SEQ ID NO: 66;
(11) a heavy chain variable region represented by the amino acid sequence of SEQ ID NO: 71 and a light chain variable region represented by the amino acid sequence of SEQ ID NO: 72;
(12) a heavy chain variable region represented by the amino acid sequence of SEQ ID NO: 77 and a light chain variable region represented by the amino acid sequence of SEQ ID NO: 78;
(13) a heavy chain variable region represented by the amino acid sequence of SEQ ID NO: 83 and a light chain variable region represented by the amino acid sequence of SEQ ID NO: 84;
(14) a heavy chain variable region represented by the amino acid sequence of SEQ ID NO: 89 and a light chain variable region represented by the amino acid sequence of SEQ ID NO: 90;
(15) a heavy chain variable region represented by the amino acid sequence of SEQ ID NO: 95 and a light chain variable region represented by the amino acid sequence of SEQ ID NO: 96; or
(16) a heavy chain variable region represented by the amino acid sequence of SEQ ID NO: 101 and a light chain variable region represented by the amino acid sequence of SEQ ID NO: 102.

In a preferred embodiment of the present invention, the antibody may be a monoclonal antibody, preferably a single-chain variable fragment (scFv).

In addition, the present invention provides a polynucleotide encoding the humanized antibody or fragment thereof that specifically binds to CD47.

In addition, the present invention provides a vector comprising a polynucleotide encoding the humanized antibody or fragment thereof that specifically binds to CD47.

In addition, the present invention provides a recombinant cell that produces a humanized antibody or fragment thereof that specifically binds to CD47, transformed with the vector.

To achieve other purposes, the present invention provides a pharmaceutical composition for preventing or treating a disease mediated by a CD47-overexpressing cell comprising the humanized antibody or fragment thereof that specifically binds to CD47.

In a preferred embodiment of the present invention, the composition may further include an immune checkpoint inhibitor, and the immune checkpoint inhibitor may preferably be an anti-PD-1 antibody.

In another preferred embodiment of the present invention, the disease mediated by the CD47-overexpressing cell may be a CD47-overexpressing cancer or tumor.

In another preferred embodiment of the present invention, the cancer or tumor may be selected from the group consisting of hematologic cancer, ovarian cancer, colon cancer, breast cancer, lung cancer, myeloma, neuroblast-derived CNS tumor, monocytic leukemia, B-cell leukemia, T- cell leukemia, B-cell lymphoma, T-cell lymphoma, and mast cell induced tumor.

### [Advantageous Effects]

In the present invention, 16 kinds of humanized antibodies were prepared using an antibody (3A5 antibody) that binds to CD47, and it was confirmed that the humanized anti-CD47 antibody specifically bound to the CD47 antigen.

In addition, the humanized anti-CD47 antibody of the present invention not only blocks CD47-SIRPα binding, but also promotes phagocytosis by macrophages by binding to cells overexpressing CD47, and inhibits the growth of CD47-expressing tumors. Therefore, it can be applied to the prevention or treatment of diseases or tumors in which the immune response by overexpression of CD47 is suppressed.

### [Description of Drawings]

FIG. 1 is data confirming the binding ability of the 3A5 (mouse) antibody selected in the present invention to CD47 overexpressing tumor cells (MCF-7) using a flow cytometer.
FIG. 2 is data confirming the binding ability of the humanized 3A5 antibody of the present invention to 16 CD47 overexpressing tumor cells (MCF-7) using a flow cytometer.
FIG. 3 is data confirming the CD47-SIRPα binding blocking ability of the humanized Hu3A5 (V10) antibody of the present invention.
FIG. 4 is data confirming that the humanized Hu3A5 (V10) antibody of the present invention promotes macrophage by binding to the surface CD47 of leukemia peripheral-derived T cells (Jurkat Cell).
FIG. 5 is data confirming (A) red blood cell and platelet binding, and (B) red blood cell aggregation level of the humanized Hu3A5(V10) antibody of the present invention and a commercial CD47 antibody (clone#CC2C6).
FIG. 6 (A) is a schematic diagram of an animal experiment method and FIG. 6 (B) is data confirming the tumor size according to administration of 3A5 antibody, when the 3A5 (mouse) antibody of the present invention is administered to a mouse transplanted with human CD47-expressing murine colon adenocarcinoma cells. As the mouse PD-1 antibody, a commercial PD-1 antibody (clone#RMP1-14) was used.
FIG. 7 (A) is a schematic diagram of an animal test method and FIG. 7 (B) is data confirming the tumor size according to administration of the Hu3A5(V10) antibody, when the humanized Hu3A5(V10) antibody of the present invention was administered to a mouse transplanted with human CD47-expressing murine colon adenocarcinoma cells (C57BL/6-hCD47/hSIRPα knock-in mouse, hCD47 KI).
FIG. 8 is data observed by immunohistochemistry (IHC) of major organs of C57BL/6-hCD47/hSIRPα knock-in mouse after administration of the Hu3A5(V10) antibody in FIG. 7.

### [Mode of the Invention]

Hereinafter, the present invention will be described in detail.

### Humanized Antibodies that Specific Binding to CD47

The present invention is from a point of view, relating to a humanized antibody or fragment thereof that specifically binds to CD47, comprising:
(1) a heavy chain variable region represented by the amino acid sequence of SEQ ID NO: 11 and a light chain variable region represented by the amino acid sequence of SEQ ID NO: 12;
(2) a heavy chain variable region represented by the amino acid sequence of SEQ ID NO: 17 and a light chain variable region represented by the amino acid sequence of SEQ ID NO: 18;
(3) a heavy chain variable region represented by the amino acid sequence of SEQ ID NO: 23 and a light chain variable region represented by the amino acid sequence of SEQ ID NO: 24;
(4) a heavy chain variable region represented by the amino acid sequence of SEQ ID NO: 29 and a light chain variable region represented by the amino acid sequence of SEQ ID NO: 30;
(5) a heavy chain variable region represented by the amino acid sequence of SEQ ID NO: 35 and a light chain variable region represented by the amino acid sequence of SEQ ID NO: 36;
(6) a heavy chain variable region represented by the amino acid sequence of SEQ ID NO: 41 and a light chain variable region represented by the amino acid sequence of SEQ ID NO: 42;
(7) a heavy chain variable region represented by the amino acid sequence of SEQ ID NO: 47 and a light chain variable region represented by the amino acid sequence of SEQ ID NO: 48;
(8) a heavy chain variable region represented by the amino acid sequence of SEQ ID NO: 53 and a light chain variable region represented by the amino acid sequence of SEQ ID NO: 54;
(9) a heavy chain variable region represented by the amino acid sequence of SEQ ID NO: 59 and a light chain variable region represented by the amino acid sequence of SEQ ID NO: 60;
(10) a heavy chain variable region represented by the amino acid sequence of SEQ ID NO: 65 and a light chain variable region represented by the amino acid sequence of SEQ ID NO: 66;
(11) a heavy chain variable region represented by the amino acid sequence of SEQ ID NO: 71 and a light chain variable region represented by the amino acid sequence of SEQ ID NO: 72;
(12) a heavy chain variable region represented by the amino acid sequence of SEQ ID NO: 77 and a light chain variable region represented by the amino acid sequence of SEQ ID NO: 78;
(13) a heavy chain variable region represented by the amino acid sequence of SEQ ID NO: 83 and a light chain variable region represented by the amino acid sequence of SEQ ID NO: 84;
(14) a heavy chain variable region represented by the amino acid sequence of SEQ ID NO: 89 and a light chain variable region represented by the amino acid sequence of SEQ ID NO: 90;
(15) a heavy chain variable region represented by the amino acid sequence of SEQ ID NO: 95 and a light chain variable region represented by the amino acid sequence of SEQ ID NO: 96; or
(16) a heavy chain variable region represented by the amino acid sequence of SEQ ID NO: 101 and a light chain variable region represented by the amino acid sequence of SEQ ID NO: 102.

In the present invention, the term "humanized antibody" refers to an antibody with increased similarity to a human antibody by making the remaining parts except for the CDR region, which is a key part for antigen binding, to an amino acid sequence corresponding to an antibody produced by humans. The most common method for humanizing antibody is a CDR-grafting method in which the CDR regions of an animal antibody are grafted into a human antibody, but is not limited thereto, and is known in the art.

In the present invention, the antibody may be a monoclonal antibody. In the present invention, the term "monoclonal antibody" is an antibody produced by a single antibody-forming cell, and has a uniform primary structure (amino acid sequence). It recognizes only one antigenic determinant, and is generally produced by culturing a hybridoma cell in which cancer cells and antibody-producing cells are fused.

As used herein, the term "CDR", i.e., "complementarity determining region", refers to a non-contiguous antigen binding site found within the variable regions of both the heavy and light chain regions.

In the present invention, the term "antibody" can be used not only in a complete form having two full-length light chains and two full-length heavy chains, but also fragments of antibody molecule. A fragment of antibody molecule means a fragment having at least a peptide tag (epitope) binding function, and includes scFv, Fab, F(ab'), F(ab')₂, a single domain, etc.

Among antibody fragments, Fab has a structure having variable regions of light and heavy chains, a constant region of light chain and the first constant region of heavy chain (CH1), and has one antigen-binding site. Fab' differs from Fab in that it has a hinge region comprising one or more cysteine residues at the C terminus of the heavy chain CH1 domain. F(ab')₂ antibody is produced by forming a disulfide bond with a cysteine residue in the hinge region of Fab'. Fv is a minimal antibody fragment having only a heavy chain variable region and a heavy chain variable region. Recombinant technology for generating an Fv fragment is described in International Patent Publications WO 88/10649, WO 88/106630, WO 88/07085, WO 88 /07086 and WO 88/09344. A double chain Fv (dsFv) has a disulfide bond, and a heavy chain variable region and a heavy chain variable region are connected, and a single chain Fv (scFv) is generally connected through a peptide linker, the variable region of the heavy chain and the variable region of the light chain are covalently bonded. Such an antibody fragment can be obtained using a proteolytic enzyme (for example, Fab can be obtained by restriction digestion of the entire antibody with papain, and F(ab')₂ fragment can be obtained by digestion with pepsin). Preferably, it can be produced through genetic recombination technology.

The monoclonal antibody that specifically binds to CD47 of the present invention can be prepared by using all or part of the CD47 protein as an immunogen (or antigen). More specifically, as an immunogen, CD47, a fusion protein containing CD47 protein, or a carrier containing CD47 protein, if necessary, together with an adjuvant (e.g., Freund adjuvant), is injected once or more by subcutaneous, intramuscular, intravenous, intraperitoneal in mammals except for humans to achieve an immunization. The mammals other than humans are preferably mice, rats, hamsters, malmots, chickens, rabbits, cats, dogs, pigs, goats, sheep, donkeys, horses or cattle (including transgenic animals engineered to produce an antibody from other animals such as mice to produce human antibody), more preferably mouse, rat, hamster, malmot, chicken or rabbit. Antibody-producing cells can be obtained from the immune-sensitized mammal about 1 to 10 days after the final immunization by performing immunization 1 to 4 times every 1 to 21 days from the first immunization. The number of times and intervals for immunization can be appropriately changed depending on the characteristics of the immunogen to be used.

Preparation of a hybridoma secreting a monoclonal antibody can be carried out according to the method of Keira and Mirstein et al. (Nature, 1975, Vol. 256, p. 495-497) and a method similar thereto. Hybridomas can be produced by cell fusion of mammal-derived myeloma cells without autologous antibody-producing ability and antibody-producing cells contained in the group consisting of spleen, lymph node, bone marrow and tonsils, preferably spleen. The mammal may be a mouse, rat, malmot, hamster, chicken, rabbit or human, preferably a mouse, rat, chicken or human.

For cell fusion, for example, a fusion promoter including polyethylene glycol or Sendai virus or a method by electric pulse is used, for example, in a fusion medium containing a fusion promoter, antibody-producing cells and mammalian-derived cells capable of indefinite proliferation. Cells are suspended at a ratio of about 1:1 to 1:10, and in this state, cultured at about 30 to 40°C for about 1 to 5 minutes. As the fusion medium, for example, MEM medium, RPMI1640 medium, and Iscove's Modified Dulbecco's Medium may be used, and it is preferable to exclude sera such as bovine serum.

In the method of screening the hybridoma clones producing the monoclonal antibody, first, the fusion cells obtained as described above are transferred to a selection medium such as HAT medium, and cultured at about 30 to 40°C. for about 3 days to 3 weeks to kill cells other than hybridomas. Then, after culturing the hybridoma on a microtiter plate, etc., the part with increased reactivity between the immunogen used for the immune response of animals other than humans described above and the culture supernatant was subjected to RIA (radioactive substance-marked immuno antibody) or ELISA (Enzyme-Linked Immunosorbent Assay). The clone producing the monoclonal antibody found above shows specific binding ability to the immunogen.

The monoclonal antibody of the present invention can be obtained by culturing such a hybridoma in vitro or in vivo. For culturing, a conventional method for culturing cells derived from mammals is used, and for collecting monoclonal antibody from a culture or the like, a conventional method in this field for purifying an antibody in general is used. As each method, for example, salting out, dialysis, filtration, concentration, centrifugation, fractional precipitation, gel filtration chromatography, ion exchange chromatography, affinity chromatography, high-performance liquid chromatography, gel electrophoresis or isoelectric point electrophoresis, etc. can be applied, and these are applied in combination as needed. The purified monoclonal antibody is then concentrated and dried to be in a liquid or solid state depending on the use.

In a specific embodiment of the present invention, in order to prepare an antibody that specifically binds to CD47, hybridomas producing an anti-CD47 antibody were prepared and screened, and then an antibody (scFv) that specifically binds to CD47 was selected and designated as 3A5.

It was confirmed that 3A5 antibody had a heavy chain variable comprising a CDR1 region represented by the amino acid of SEQ ID NO: 1 (GYTFTSYW), a CDR2 region represented by the amino acid of SEQ ID NO: 2 (IDPSDSYT) and a CDR3 region represented by the amino acid of SEQ ID NO: 3 (ARGGKRAMDY) and a light chain variable region comprising a region and a CDR1 region represented by the amino acid of SEQ ID NO: 4 (QSLVHSNGNTY), a CDR2 region represented by the amino acid of SEQ ID NO: 5 (KVS) and a CDR3 region represented by the amino acid of SEQ ID NO: 6 (SQSTHVPFT).

Specifically, 3A5 antibody contained a heavy chain variable region represented by the amino acid of SEQ ID NO: 7 and a light chain variable region represented by the amino acid of SEQ ID NO: 8, wherein the heavy chain variable region was encoded with the nucleotide sequence of SEQ ID NO: 9, and the light chain variable region was encoded with the nucleotide sequence of SEQ ID NO: 10.

In another specific embodiment of the present invention, 16 kinds of humanized antibodies were prepared in which the anti-CD47 antibody 3A5 was changed to a structure corresponding to humans, which were named as Hu3A5 (V1), Hu3A5 (V2), Hu3A5 (V3), Hu3A5 (V4), Hu3A5 (V5), Hu3A5 (V6), Hu3A5 (V7), Hu3A5 (V8), Hu3A5 (V9), Hu3A5 (V10), Hu3A5 (V11), Hu3A5 (V12), Hu3A5 (V13), Hu3A5 (V14), Hu3A5 (V15) and Hu3A5 (V16).

The heavy chain variable region CDRs and the light chain variable region CDRs of the 16 humanized anti-CD47 antibodies were the same as those of 3A5, except for the CDR regions, that were humanized.

Preferably, Hu3A5(V1) antibody has a heavy chain variable region represented by the amino acid sequence of SEQ ID NO: 11 and a light chain variable region represented by the amino acid sequence of SEQ ID NO: 12, wherein the heavy chain variable region may be encoded by the nucleotide sequence of SEQ ID NO: 13 and the light chain variable region may be encoded by the nucleotide sequence of SEQ ID NO: 14.

Hu3A5(V2) antibody has a heavy chain variable region represented by the amino acid sequence of SEQ ID NO: 17 and a light chain variable region represented by the amino acid sequence of SEQ ID NO: 18, wherein the heavy chain variable region may be encoded by the nucleotide sequence of SEQ ID NO: 19 and the light chain variable region may be encoded by the nucleotide sequence of SEQ ID NO: 20.

HU3A5 (V3) antibody has a heavy chain variable region represented by the amino acid sequence of SEQ ID NO: 23 and a light chain variable region represented by the amino acid sequence of SEQ ID NO: 24, wherein the heavy chain variable region may be encoded by the nucleotide sequence of SEQ ID NO: 25 and the light chain variable region may be encoded by the nucleotide sequence of SEQ ID NO: 26.

Hu3A5 (V4) antibody has a heavy chain variable region represented by the amino acid sequence of SEQ ID NO: 29 and a light chain variable region represented by the amino acid sequence of SEQ ID NO: 30, wherein the heavy chain variable region may be encoded by the nucleotide sequence of SEQ ID NO: 31 and the light chain variable region may be encoded by the nucleotide sequence of SEQ ID NO: 32.

Hu3A5 (V5) antibody has a heavy chain variable region represented by the amino acid sequence of SEQ ID NO: 35 and a light chain variable region represented by the amino acid sequence of SEQ ID NO: 36, wherein the heavy chain variable region may be encoded by the nucleotide sequence of SEQ ID NO: 37 and the light chain variable region may be encoded by the nucleotide sequence of SEQ ID NO: 38.

Hu3A5(V6) antibody has a heavy chain variable region represented by the amino acid sequence of SEQ ID NO: 41 and a light chain variable region represented by the amino acid sequence of SEQ ID NO: 42, wherein the heavy chain variable region may be encoded by the nucleotide sequence of SEQ ID NO: 43 and the light chain variable region may be encoded by the nucleotide sequence of SEQ ID NO: 44.

Hu3A5(V7) antibody has a heavy chain variable region represented by the amino acid sequence of SEQ ID NO: 47 and a light chain variable region represented by the amino acid sequence of SEQ ID NO: 48, wherein the heavy chain variable region may be encoded by the nucleotide sequence of SEQ ID NO: 49 and the light chain variable region may be encoded by the nucleotide sequence of SEQ ID NO: 50.

Hu3A5 (V8) antibody has a heavy chain variable region represented by the amino acid sequence of SEQ ID NO: 53 and a light chain variable region represented by the amino acid sequence of SEQ ID NO: 54, wherein the heavy chain variable region may be encoded by the nucleotide sequence of SEQ ID NO: 55 and the light chain variable region may be encoded by the nucleotide sequence of SEQ ID NO: 56.

Hu3A5(V9) antibody has a heavy chain variable region represented by the amino acid sequence of SEQ ID NO: 59 and a light chain variable region represented by the amino acid sequence of SEQ ID NO: 60, wherein the heavy chain variable region may be encoded by the nucleotide sequence of SEQ ID NO: 61 and the light chain variable region may be encoded by the nucleotide sequence of SEQ ID NO: 62.

Hu3A5(V10) antibody has a heavy chain variable region represented by the amino acid sequence of SEQ ID NO: 65 and a light chain variable region represented by the amino acid sequence of SEQ ID NO: 66, wherein the heavy chain variable region may be encoded by the nucleotide sequence of SEQ ID NO: 67 and the light chain variable region may be encoded by the nucleotide sequence of SEQ ID NO: 68.

Hu3A5(V11) antibody has a heavy chain variable region represented by the amino acid sequence of SEQ ID NO: 71 and a light chain variable region represented by the amino acid sequence of SEQ ID NO: 72, wherein the heavy chain variable region may be encoded by the nucleotide sequence of SEQ ID NO: 73 and the light chain variable region may be encoded by the nucleotide sequence of SEQ ID NO: 74.

Hu3A5(V12) antibody has a heavy chain variable region represented by the amino acid sequence of SEQ ID NO: 77 and a light chain variable region represented by the amino acid sequence of SEQ ID NO: 78, wherein the heavy chain variable region may be encoded by the nucleotide sequence of SEQ ID NO: 79 and the light chain variable region may be encoded by the nucleotide sequence of SEQ ID NO: 80.

Hu3A5(V13) antibody has a heavy chain variable region represented by the amino acid sequence of SEQ ID NO: 83 and a light chain variable region represented by the amino acid sequence of SEQ ID NO: 84, wherein the heavy chain variable region may be encoded by the nucleotide sequence of SEQ ID NO: 85 and the light chain variable region may be encoded by the nucleotide sequence of SEQ ID NO: 86.

Hu3A5(V14) antibody has a heavy chain variable region represented by the amino acid sequence of SEQ ID NO: 89 and a light chain variable region represented by the amino acid sequence of SEQ ID NO: 90, wherein the heavy chain variable region may be encoded by the nucleotide sequence of SEQ ID NO: 91 and the light chain variable region may be encoded by the nucleotide sequence of SEQ ID NO: 92.

Hu3A5(V15) antibody has a heavy chain variable region represented by the amino acid sequence of SEQ ID NO: 95 and a light chain variable region represented by the amino acid sequence of SEQ ID NO: 96, wherein the heavy chain variable region may be encoded by the nucleotide sequence of SEQ ID NO: 97 and the light chain variable region may be encoded by the nucleotide sequence of SEQ ID NO: 98.

Hu3A5(V16) antibody has a heavy chain variable region represented by the amino acid sequence of SEQ ID NO: 101 and a light chain variable region represented by the amino acid sequence of SEQ ID NO: 102, wherein the heavy chain variable region may be encoded by the nucleotide sequence of SEQ ID NO: 103 and the light chain variable region may be encoded by the nucleotide sequence of SEQ ID NO: 104.

The CD47-specific antibody of the present invention is preferably scFv (single chain variable fragment), and can be produced through genetic recombination technology so that the heavy chain variable region and the light chain variable region can be linked with a linker. The linker is preferably represented by the amino acid sequence of SEQ ID NO: 107 or may be encoded by the nucleotide sequence of SEQ ID NO: 108 to SEQ ID NO: 123, but is not limited thereto.

When linked by a light chain variable region-linker-heavy chain variable region, Hu3A5 (V1) antibody may has the amino acid sequence of SEQ ID NO: 15 or the nucleotide sequence of SEQ ID NO: 16; Hu3A5 (V2) antibody may has the amino acid sequence of SEQ ID NO: 21 or the nucleotide sequence of SEQ ID NO: 22; 3A5 (V3) antibody may has the amino acid sequence of SEQ ID NO: 27 or the nucleotide sequence of SEQ ID NO: 28; Hu3A5 (V4) antibody may has the amino acid sequence of SEQ ID NO: 33 or the nucleotide sequence of SEQ ID NO: 34; Hu3A5 (V5) antibody may has the amino acid sequence of SEQ ID NO: 39 or the nucleotide sequence of SEQ ID NO: 40; Hu3A5 (V6) antibody may has the amino acid sequence of SEQ ID NO: 45 or the nucleotide sequence of SEQ ID NO: 46; Hu3A5 (V7) antibody may has the amino acid sequence of SEQ ID NO: 51 or the nucleotide sequence of SEQ ID NO: 52; 3A5 (V8) antibody may has the amino acid sequence of SEQ ID NO: 57 or nucleotide sequence of SEQ ID NO: 58; Hu3A5 (V9) antibody may has the amino acid sequence of SEQ ID NO: 63 or nucleotide sequence of SEQ ID NO: 64; Hu3A5 (V10) antibody may has the amino acid sequence of SEQ ID NO: 69 or the nucleotide sequence of SEQ ID NO: 70; Hu3A5 (V11) antibody may has the amino acid sequence of SEQ ID NO: 75 or the nucleotide sequence of SEQ ID NO: 76; Hu3A5 (V12) antibody may has the amino acid sequence of SEQ ID NO: 81 or the nucleotide sequence of SEQ ID NO: 82; Hu3A5 (V13) antibody may has the amino acid sequence of SEQ ID NO: 87 or the nucleotide sequence of SEQ ID NO: 88; Hu3A5 (V14) antibody may has the amino acid sequence of SEQ ID NO: 93 or the nucleotide sequence of No. 94; Hu3A5 (V15) antibody may has the amino acid sequence of SEQ ID NO: 99 or the nucleotide sequence of SEQ ID NO: 100; and Hu3A5 (V16) antibody may has the amino acid sequence of SEQ ID NO: 105 or the nucleotide sequence of SEQ ID NO: 106.

In the present invention, the antibody can prevent CD47 from interacting with signal-regulating-protein (SIRPα) or promote macrophage-mediated phagocytosis on CD47-expressing cells.

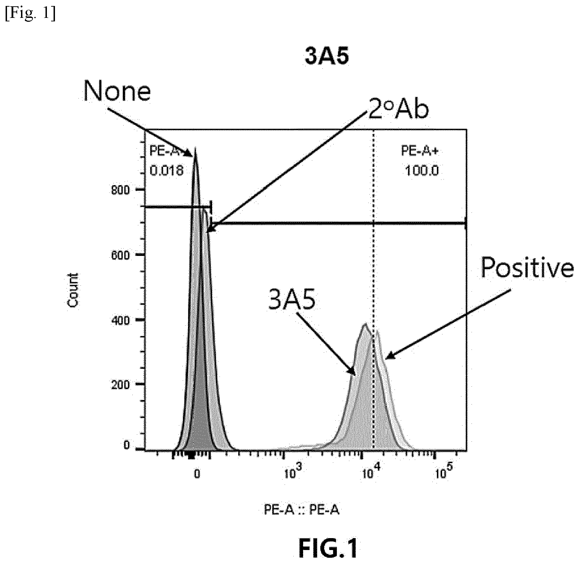

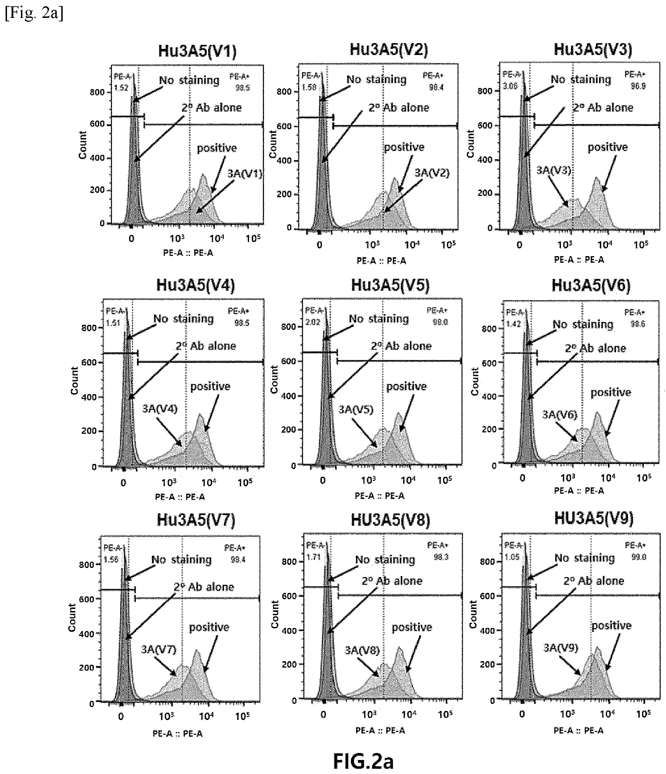

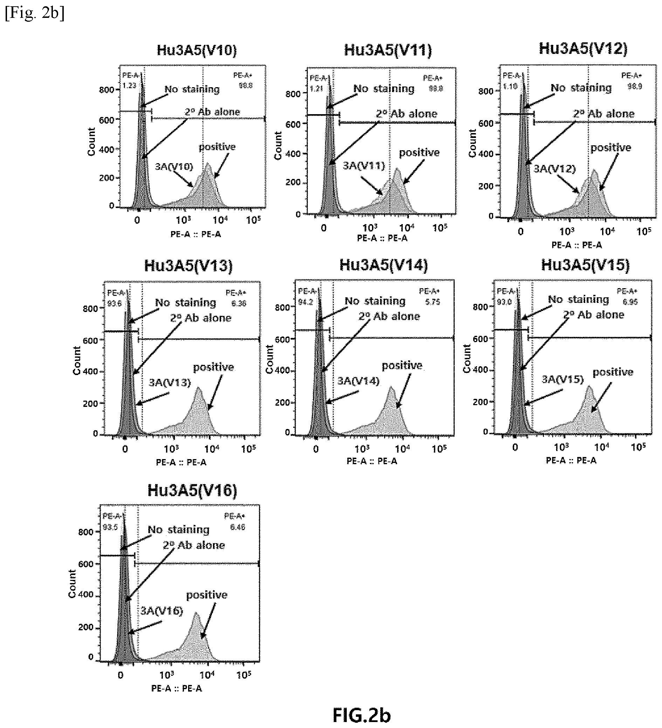

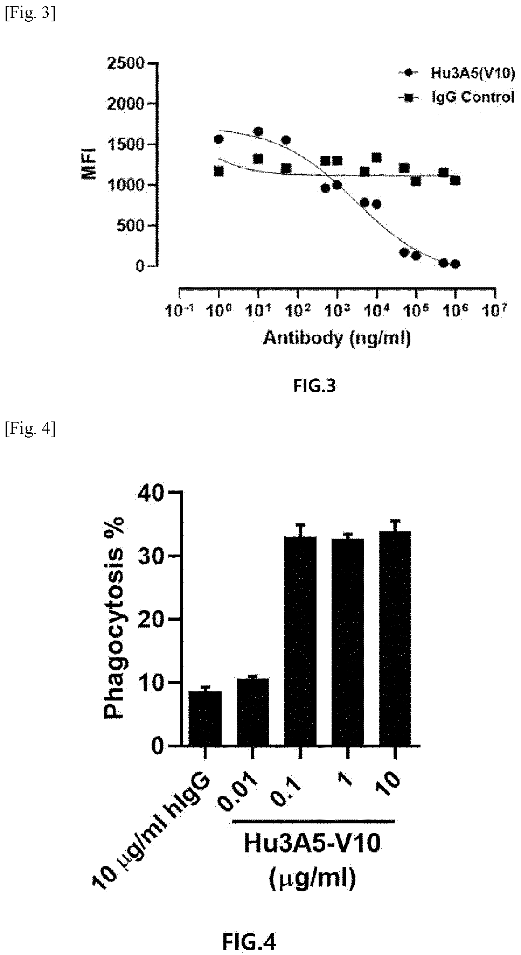

In a specific embodiment of the present invention, it was confirmed that all of 3A5 antibody selected in the present invention and 16 kinds of humanized Hu3A5 antibodies thereof specifically bound to CD47-overexpressing cells (FIGS. 1 and 2). In addition, as a result of confirming whether the humanized Hu3A5(V10) antibody of the present invention prevents the interaction between CD47 and SIRPα, it was confirmed that it effectively blocked CD47-SIRPα binding as shown in FIG. 3.

In another specific embodiment of the present invention, in order to confirm that the humanized 3A5 antibody can actually be used as an antibody therapeutic agent, Hu3A5 (V10) antibody was treated with leukemia peripheral type-derived T cells (Jurkat cells). As a result, it was confirmed that phagocytosis of Jurkat cells by macrophages was effectively induced by Hu3A5 (V10).

CD47 is also present in large amounts on the surface of red blood cells, and when the administered anti-CD47 antibody attaches to red blood cells, macrophages eat red blood cells, resulting in anemia or hemagglutination. Adverse effects due to erythrocyte phagocytosis have been reported for some commercially available CD47 antibodies or in clinical trials.

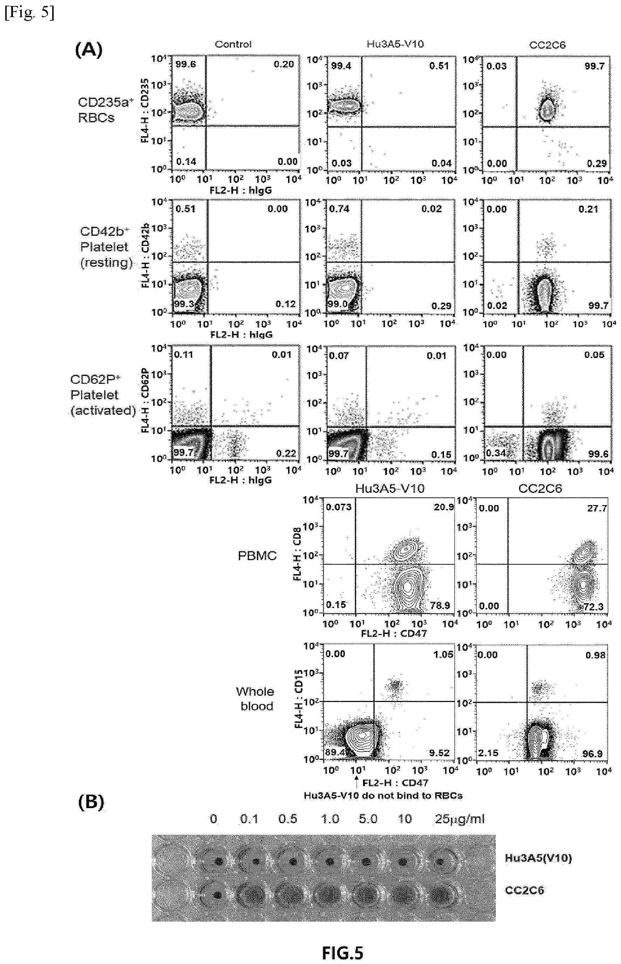

In another specific embodiment of the present invention, in order to determine whether the humanized 3A5 antibody induces a hemagglutination reaction, it was checked whether or not there was a hemagglutination reaction or they would bind to red blood cells/platelets to the humanized Hu3A5 (V10) antibody and a commercial anti-CD47 antibody (clone#CC2C6). As shown in Fig. 5, the commercial anti-CD47 antibody (clone#CC2C6) reacted with erythrocytes to induce hemagglutination, whereas the humanized Hu3A5 (V10) antibody of the present invention did not bind erythrocytes and platelets (Fig. 5A), and did not induce hemagglutination (Fig. 5B).

That is, the humanized anti-CD47 antibody of the present invention specifically recognized cells overexpressing CD47, and blocked CD47-SIRPα binding to suppress immune evasion of cancer or tumor cells, as well as effectively promoted the phagocytosis of cancer cells overexpressing CD47 by macrophages. Furthermore, since the humanized anti-CD47 antibody does not induce hemagglutination, it can be used as an antibody therapeutic for the prevention or treatment of cancer or tumors overexpressing CD47 more safely and effectively.

In another aspect, the present invention relates to a polynucleotide encoding an antibody that specifically binds to CD47.

As used herein, the term "polynucleotide" generally refers to a nucleic acid molecule, deoxyribonucleotide or ribonucleotide, or an analog thereof, separated by any length. In some embodiments, a polynucleotide of the present invention can be prepared by (1) in-vitro amplification, such as polymerase chain reaction (PCR) amplification; (2) cloning and recombination; (3) purification such as digestion and gel electrophoretic separation; (4) synthesis such as chemical synthesis, and preferably, the isolated polynucleotide is prepared by recombinant DNA technology. In the present invention, the nucleic acid for encoding the antibody or antigen-binding fragment thereof can be prepared by various methods known in the art, including, but not limited to, restriction fragment operation of synthetic oligonucleotides or application of SOE PCR.

In another aspect, the present invention relates to a vector comprising the polynucleotide encoding the antibody that specifically binds to CD47, and a recombinant cell transformed with the vector.

In the present invention, the term "vector (expression vector)" refers to a gene preparation including essential regulatory elements such as a promoter so that a target gene can be expressed in an appropriate host cell. A vector may be selected from one or more of a plasmid, a retroviral vector, and a lentiviral vector. Upon transformation into an appropriate host, a vector can replicate and function independently of the host genome, or in some cases can be integrated into the genome itself.

In addition, a vector may contain expression control elements that allow the coding region to be accurately expressed in a suitable host. Such regulatory elements are well known to those skilled in the art and include, for example, promoters, ribosome-binding sites, enhancers and other regulatory elements for regulating gene transcription or mRNA translation. The specific structure of the expression control sequence may vary depending on the function of the species or cell type, but generally contains 5' non-translated sequence, and a 5' or 3' non-translated sequence participating in transcription initiation and translation initiation, respectively, such as TATA box, capped sequence, CAAT sequence, etc. For example, a 5' non-transcriptional expression control sequence can include a promoter region that can include a promoter sequence for transcription and control of a functionally linked nucleic acid.

As used herein, the term "promoter" means a minimal sequence sufficient to direct transcription. In addition, promoter constructs sufficient to allow expression of a regulatable promoter-dependent gene induced by cell type-specific or external signals or agents may be included, and these constructs may be located in the 5' or 3' portion of the gene. Both conservative and inducible promoters are included. Promoter sequences may be derived from prokaryotes, eukaryotes or viruses.

In the present invention, the term "transformant" refers to a cell transformed by introducing a vector having a polynucleotide encoding one or more target proteins into a host cell, and a method for introducing the expression a vector into the host cell to form a transformant are such as a calcium phosphate method or a calcium chloride/rubidium chloride method, an electroporation method, an electroinjection method, a chemical treatment method such as PEG, a method using a gene gun, and the like (Sambrook, J., et al., Molecular Cloning, A Laboratory Manual(2nd ed.), Cold Spring Harbor Laboratory, 1. 74, 1989).

When the transformant expressing the vector is cultured in a nutrient medium, an antibody protein can be produced and isolated in large quantities. Medium and culture conditions can be appropriately selected and used depending on the host cell. During culture, conditions such as temperature, medium pH, and culture time should be appropriately adjusted to be suitable for cell growth and mass production of proteins.

The vector according to the present invention can be transformed into a host cell, preferably a mammalian cell, for the production of the antibody. Suitable host cells capable of expressing fully glycosylated proteins include COS-1 (e.g. ATCC CRL 1650), COS-7 (e.g. ATCC CRL-1651), HEK293, BHK21 (e.g. ATCC CRL-10), CHO (e.g. ATCC CRL 1610) and BSC-1 (e.g. ATCC CRL-26) cell lines, Cos-7 cells, CHO cells, hep G2 cells, P3X63Ag8.653, SP2/0 -Agl4, 293 cells, HeLa cells, etc., and these cells are readily available from, for example, ATCC (American Type Culture Collection, USA).

### Composition for preventing or treating diseases mediated by CD47 overexpression

In another aspect, the present invention relates to a pharmaceutical composition for preventing or treating a disease mediated by CD47 overexpression, comprising a humanized antibody that specifically binds to CD47.

In the present invention, the disease mediated by CD47 overexpression may be a cancer or tumor overexpressing CD47, preferably, the cancer or tumor overexpressing CD47 can be selected from the group consisting of blood cancer, ovarian cancer, colon cancer, breast cancer, lung cancer, myeloma, neuroblast-derived CNS tumors, monocyte leukemia, B-cell leukemia, T-cell leukemia, B-cell lymphoma, T-cell lymphoma, and mast cell induced tumor.

In the present invention, the composition may further include a therapeutic agent for a disease mediated by cells overexpressing CD47, wherein the therapeutic agent is covalently bound to the heavy and/or light chain of an antibody that specifically binds to CD47. The therapeutic agent can be administered in combination with the humanized antibody specific for CD47 of the present invention.

The therapeutic agent includes a small molecule drug, a peptide drug, a toxin (e.g., a cytotoxin), and the like. In addition, the therapeutic agent may be an anticancer agent. Anticancer agents reduce the proliferation of cancer cells and include non-peptidyl (i.e., non-protein) compounds, including cytotoxic agents and cytostatic agents. Non-limiting examples of anticancer agents include alkylating agents, nitrosourea, antimetabolites, antitumor antibiotics, plant (vinca) alkaloids, and steroid hormones. Peptide compounds may also be used.

In addition, the therapeutic agent added to the composition may preferably be an immune checkpoint inhibitor, more preferably an anti-PD-1 antibody.

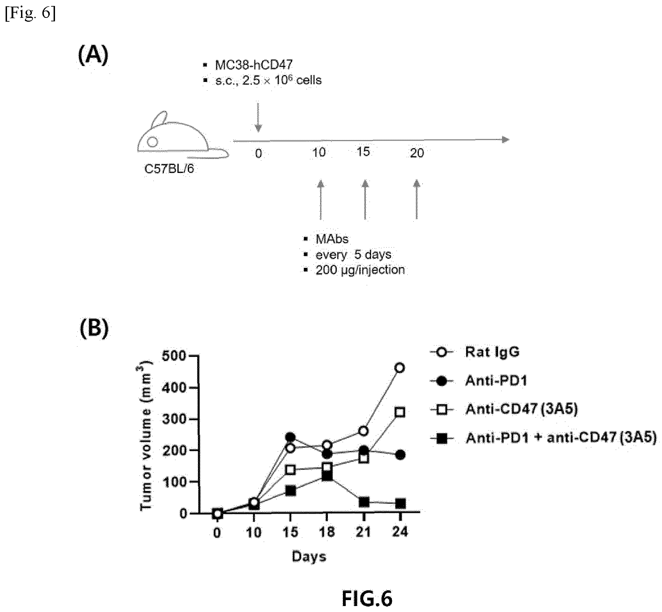

In a preferred embodiment of the present invention, in order to determine whether 3A5 antibody inhibits the growth of CD47-expressing tumors, as shown in the schematic diagram of FIG. 6A, CD47-expressing murine colon adenocarcinoma cells (murine colon adenocarcinoma cells, MC38-hCD47) was transplanted into mice, followed by control antibody (Rat IgG), anti-PD-1 antibody, anti-CD47 antibody (3A5 antibody), and anti-PD-1 antibody (clone#RMP1-14) + anti-CD47 antibody (3A5 antibody) were administered respectively. As a result, as shown in FIG. 6B, it was confirmed that the growth of tumors overexpressing CD47 was inhibited in the 3A5 antibody alone group, and in particular, the tumor growth inhibitory effect of the 3A5 antibody and anti-PD-1 antibody combination group was the best. This means that when the anti-PD-1 antibody, which is an immune checkpoint inhibitor, and the 3A5 antibody of the present invention are co-administered, a synergistic effect on tumor treatment is exhibited.

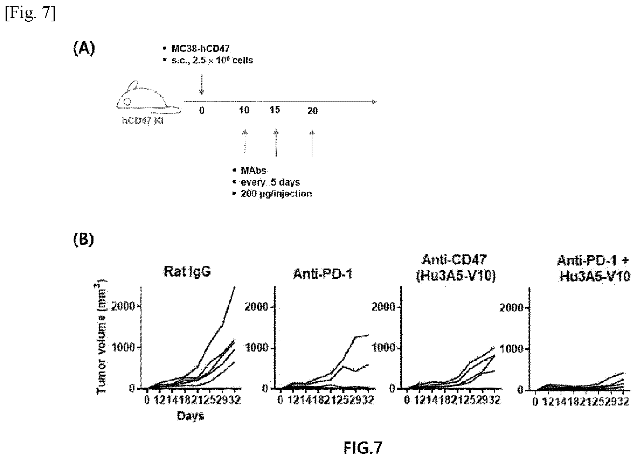

In another preferred embodiment of the present invention, in order to confirm the tumor growth inhibitory efficacy of the humanized 3A5 antibody, after transplantation of CD47-expressing murine colon adenocarcinoma cells expressing human CD47 to C57BL/6-hCD47/hSIRPα knock-in mouse where the mouse CD47 and SIRPα genes were replaced with human CD47 and human SIRPα genes, followed by control antibody (Rat IgG), anti-PD-1 antibody (clone#RMP1-14), anti-CD47 antibody (Hu3A5(V10) antibody), and anti-PD-1 antibody (clone#RMP1-14) + anti-CD47 antibody (Hu3A5(V10) antibody) were administered, respectively (FIG. 7A). As a result, it was confirmed that tumor growth was inhibited in the Hu3A5(V10) antibody alone group, and in particular, it was confirmed that the tumor growth inhibitory effect of the Hu3A5(V10) antibody and anti-PD-1 antibody combination group was the best.

In addition, after administration of the Hu3A5 (V10) antibody, major organs of C57BL/6-hCD47/hSIRPα knock-in mouse were observed by immunohistochemistry (IHC). As a result, major tissue damage due to inflammation was not observed.

That is, the humanized Hu3A5 antibody of the present invention can inhibit the growth of a tumor by targeting a cancer or tumor that overexpresses CD47 without damaging other tissues. It was confirmed that it can be applied for use in preventing or treating a cancer or tumor expressing CD47.

The pharmaceutical composition preferably contains a therapeutically effective amount of antibody of the present invention. As used herein, the term "therapeutically effective amount" refers to an amount of a therapeutic agent required to treat, ameliorate, or prevent a target disease or condition, or the amount of a therapeutic agent required to exhibit a detectable therapeutic or prophylactic effect. For any antibody, a therapeutically effective dose can be initially determined by cell culture assays or animal models, usually rodents, rabbits, dogs, pigs, or primates. Animal models can also be used to determine appropriate concentration ranges and routes of administration. Such information can be used to determine useful dosages and routes for dosing in humans.

The precise effective amount for a human patient can vary depending on the severity of the disease state, the patient's general health, the patient's age, weight and sex, diet, administration time, administration frequency, drug composition, response sensitivity, and tolerance/response to treatment. The amount can be determined by routine experimentation and is within the scope of the clinician's judgment. In general, an effective dosage is 0.01-50 mg/kg, preferably 0.1-20 mg/kg, more preferably about 15 mg/kg.

The compositions may be administered to the patient individually or in combination with other preparations, agents, or hormones. The dosage at which the antibody of the present invention is administered depends on the nature of the condition to be treated, the grade of malignant lymphoma or leukemia, and whether the antibody is used to prevent disease or to treat an existing condition.

The frequency of administration depends on the half-life of the antibody molecule and the duration of the drug's effect. If an antibody molecule has a short half-life (e.g., 2 to 10 hours), it may be necessary to provide one or more doses per day. Alternatively, if an antibody molecule has a long half-life (e.g., 2 to 15 days), it may be necessary to provide a dose once a day, once a week, or once every 1 or 2 months.

In addition, the pharmaceutical composition may contain a pharmaceutically acceptable carrier for administration of an antibody. The carrier itself must not cause the production of an antibody that is harmful to the subject receiving the composition, and must be non-toxic. Suitable carriers may be slowly metabolized macromolecules, such as proteins, polypeptides, liposomes, polysaccharides, polylactic acid, polyglycolic acid, amino acid polymers, amino acid copolymers and inactive viral particles.

A pharmaceutically acceptable salt may be used, and it contains for example, mineral acid salts such as hydrochloride, hydrobromide, phosphate and sulfate, or salts of organic acids such as acetic acid, propionic acid, malonic acid and benzoic acid.

A pharmaceutically acceptable carrier in therapeutic compositions may additionally include liquids such as water, saline, glycerol and ethanol. Additionally, auxiliary substances such as wetting agents, emulsifying agents or pH buffering agents may be present in such compositions. The carrier may be formulated as tablets, pills, sugar-coated tablets, capsules, liquids, gels, syrups, slurries and suspensions for ingestion of the pharmaceutical composition by a patient.

Preferred forms for administration may include those suitable for parenteral administration, for example by injection or infusion. When the product is intended for infusion or injection, it may take the form of suspensions, solutions or emulsions in oil or water-soluble excipients, which may contain prescription agents such as suspending, preservative, stabilizing and/or dispersing agents. Alternatively, an antibody molecule may be in anhydrous form and reconstituted with an appropriate sterile solution prior to use.

Once formulated, the compositions of the present invention can be administered directly to a patient. The patients to be treated may be animals. However, the composition is preferably adapted for administration to human patients.

The pharmaceutical composition of the present invention is not limited, but oral, intravenous, intramuscular, intraarterial, intramedullary, intrathecal, intraventricular, transdermal, transcutaneous, subcutaneous, intraperitoneal, intranasal, intestinal, topical, sublingual, intravaginal or rectal routes. Typically, the therapeutic composition may be prepared as injectable forms as liquid solutions or suspensions. In addition, solid forms suitable for solution or suspension in liquid excipients prior to injection may be prepared.

Direct delivery of the composition may generally be achieved by injection, subcutaneous injection, intraperitoneal injection, intravenous injection, intramuscular injection, or may be delivered to the interstitial space of a tissue. In addition, the composition may be administered to the wound site. Dosage treatment may be a single dose schedule or a multiple dose schedule.

### Diagnosis or monitoring of diseases mediated by cells expressing CD47

In another aspect, the present invention relates to a composition for diagnosing or monitoring a disease mediated by cells expressing CD47, including the antibody that specifically binds to CD47.

The antibody that specifically binds to CD47 may be directly or indirectly labeled. Indirect labels include secondary antibodies comprising a detectable label, wherein the secondary antibody binds to an antibody that specifically binds to CD47. Other indirect labels include biotin, wherein an antibody that specifically binds to biotinylated CD47 can be detected using avidin or streptavidin comprising a detectable label.

A suitable detectable label includes any composition detectable by spectroscopic, photochemical, biochemical, immunochemical, electrical, optical or chemical means. A suitable labels includes, but are not limited to, magnetic beads, fluorescent dyes (e.g., fluorescein isothiocyanate, Texas red, rhodamine, green fluorescent protein, red fluorescent protein, yellow fluorescent protein, etc.), radioactive labels (e.g., For example, ³H, ¹²⁵I, ³⁵S, ¹⁴C or ³²P), enzymes (e.g., mustard radish peroxidase, alkaline phosphatase, luciferase and the ones commonly used for enzyme-linked immunosorbent assay (ELISA)) and colorimetric labels such as colloidal gold or tinted glass or plastic (e.g., polystyrene, polypropylene, latex, etc.) beads.

In addition, for diagnosis or monitoring, the antibody may be labeled with a fluorescent protein, and may contain a contrast agent or a radioisotope.

When the antibody that specifically binds to CD47 of the present invention is used in a diagnostic kit, the antibody is immobilized on a support, and the support may be a microplate, microarray, chip, glass, bead or particle, or a membrane.

Hereinafter, preferred examples are presented to help the understanding of the present invention. However, the following examples are only provided for easier understanding of the present invention, and the contents of the present invention are not limited by the following examples.

### Example 1: Preparation and selection of antibodies that specifically bind to CD47

In order to select the CD47 peptide-specific antibody, a hybridoma producing an antibody binding to CD47 was prepared and the antibody was selected.

First, splenocytes were extracted by immunization with CD47 protein (Acrobiosystems, cat#CD7-HA2E9), and hybridoma cells were prepared through cell fusion with mouse myeloma cells.

Since mouse myeloma cells used for cell fusion do not have HGPRT (Hypoxanthine Guanidine-Phosphoribosyl-Transferase), they cannot survive in HAT medium, but hybridomas can survive in HAT medium by fusion with splenocytes. Since only hybridomas can be grown using this, it is usually grown in HAT medium until hybridomas are established.

The limiting dilution method was used to select hybridomas producing an antibody binding to CD47 from among the proliferated hybridomas. First, it was made to be less than one cell per 96 well, and then, it was confirmed by ELISA whether the antibody obtained from the clones proliferated from one cell binds to CD47, and clones that bind to CD47 were selected. The above process was repeated three times to select hybridomas producing an antibody binding to CD47. In this way, an antibody binding to CD47 was obtained.

The antibody was named 3A5, and its base and amino acid sequences were analyzed. The sequence information on the heavy chain variable region and the light chain variable region of each antibody according to the sequencing results is shown in Table 1, and the underlined parts in Table 1 mean the complementarity determining region (CDR).

**[Table 1]**

| Sequence information of 3A5 antibody | | |
|---|---|---|
| 3A5 | sequence information | SEQ ID NO: |
| Heavy chain variable region CDR1 | GYTFSYW | SEQ ID NO: 1 |
| Heavy chain variable region CDR2 | IDPSDSYT | SEQ ID NO: 2 |
| Heavy chain variable region CDR3 | ARGGKRAMDY | SEQ ID NO: 3 |
| Light chain variable region CDR1 | QSLVHSNGNTY | SEQ ID NO: 4 |
| Light chain variable region CDR2 | KVS | SEQ ID NO: 5 |
| Light chain variable region CDR3 | SQSTHVPFT | SEQ ID NO: 6 |
| amino acid sequence of heavy chain variable region | EVQLQQPGAELVKPGASVKMSCKASGYTFTSYWMHWMN QRPGQGLEWIGVIDPSDSYTSYNQKFKGKATLTVDTSSSTA YMQLSSLTSEDSAVYYCARGGKRAMDY WGQG TSVTVSS | SEQ ID NO: 7 |
| amino acid sequence of light chain | DVLMTQTPLSLPVSLGDQASISCRSSQSLVHSNGNTYLHWY LQKPGQSPKLLIYKVSNRFSGVPDRFSGSGSGTDFTLKISRVE AEDLGVYFC SQSTHVPFT FGSGTKLEIK | SEQ ID NO: 8 |
| variable region | | |
| nucleotide sequence of heavy chain variable region | GAGGTCCAGCTGCAGCAGCCTGGGGCTGAGCTGGTGAAG CCTGGGGCTTCAGTGAAGATGTCCTGCAAGGCTTCTGGCT ACACCTTCACCAGCTACTGGATGCACTGGATGAACCAGA GGCCTGGACAAGGCCTTGAGTGGATCGGAGTGATTGATC CTTCTGATAGTTATACTAGCTACAATCAAAAGTTCAAGGG CAAGGCCACATTGACTGTAGACACATCCTCCAGCACAGC CTACATGCAGCTCAGCAGCCTGACATCTGAGGACTCTGCG GTCTATTACTGTGCAAGAGGGGGTAAGAGAGCTATGGAC TACTGGGGTCAAGGAACCTCAGTCACCGTCTCCTCA | SEQ ID NO: 9 |
| nucleotide sequence of light chain variable region | GATGTTTTGATGACCCAAACTCCACTCTCCCTGCCTGTCA GTCTTGGAGATCAAGCCTCCATCTCTTGCAGATCTAGTCA GAGCCTTGTACACAGTAATGGAAACACCTATTTACATTGG TACCTGCAGAAGCCAGGCCAGTCTCCAAAGCTCCTGATCT ACAAAGTTTCCAACCGATTTTCTGGGGTCCCAGACAGGTT CAGTGGCAGTGGATCAGGGACAGATTTCACACTCAAGAT CAGCAGAGTGGAGGCTGAGGATCTGGGAGTTTATTTCTG CTCTCAAAGTACACATGTTCCATTCACGTTCGGCTCGGGG ACAAAGTTGGAAATAAAA | SEQ ID NO: 10 |

### Example 2: Confirmation of specificity of the selected antibody for CD47 - ELISA and flow cytometer

### 2-1: ELISA analysis

In the present invention, in order to confirm the specificity of 3A5 antibody established in Example 1 for CD47, ELISA analysis was performed.

First, in order to encode the CD47 peptide, the CD47 protein (Acrobiosystems, cat#CD7-HA2E9) was dispensed in a 96-well plate at a concentration of 100 ng/well, and then reacted at 4°C overnight. Then, after treatment with 1 X PBST containing 3% BSA, blocking at room temperature for 30 minutes.

3 µl of the hybridoma cell culture solution of each clone producing the 3A5 antibody was treated in each well, reacted at room temperature for 2 hours, and then washed 3 times with 1 X PBST. Secondary antibody (anti-HRP, 1: 10,000) was treated and reacted at room temperature for 30 minutes, washed 3 times with 1 X PBST, and then treated with TMB for color development and reacted at room temperature for 5 minutes. Finally, the reaction was terminated by treatment with a stop solution of 1N H₂SO₄, and then the absorbance was measured at 450 nm.

**[Table 2]**

| ELISA Experimental Conditions | |
|---|---|
| ELISA reader | Infinite F50 |
| Measurement Filter | 450 nm |
| Measurement Mode | Single Point Photo |
| Antigen Coating | 100 ng/well |
| 2nd Antibody (Anti-mIgG-HRP) | 1:10,000 dilution |
| Substrate | TMB |

**[Table 3]**

| ELISA test results | |
|---|---|
| Antibody type | OD450 measurements |
| 3A5 | 2.010 |

As a result, as shown in Table 3, it was confirmed that the 3A5 antibody selected in the present invention specifically bound to CD47.

### 2-2: Analysis using a flow cytometer

In the present invention, in order to confirm the specificity of 3A5 antibody established in Example 1 for CD47, flow cytometer was performed.

First, the CD47-overexpressing breast cancer cell line MCF-7 (1 × 10⁷) and 3A5 antibody (1 µg) were reacted for 30 minutes, and then the surface was stained with a secondary antibody, followed by measurement by flow cytometry.

As a positive control, a CD47 antibody (Biolegend PE anti-human CD47, cat# 323108, 5 µl) was used, and as a secondary antibody, a PE-conjugated goat anti-mouse IgG antibody (PE-conjugated goat anti-mouse IgG; Biolegend Inc., cat# 405307, USA, 5 µl) was used.

**[Table 4]**

| Flow cytometry results | | | |
|---|---|---|---|
| Antibody type | Count | Median | Mean |
| 3A5 | 11285 | 13866 | 14951 |
| positive | 11535 | 10972 | 11930 |
| 2nd Ab alone | 11285 | 73.9 | 80 |
| none | 11077 | 24.5 | 25.0 |

As a result, as shown in FIG. 1 and Table 4, it was confirmed that the 3A5 antibody specifically bound to cells overexpressing CD47.

### Example 3: Preparation of humanized antibody based on 3A5 antibody

A humanized antibody was prepared in which the 3A5 antibody selected in Example 1 was changed to a structure corresponding to a human.

Specifically, a mouse 3A5 antibody by a CDR-grafting method that replaces the CDRs of a human antibody with the CDRs of a mouse antibody that binds to CD47 using the germline sequence of a human antibody as a frame. 16 kinds of humanized antibodies were prepared. Humanized antibodies were named as Hu3A5 (V1), Hu3A5 (V2), Hu3A5 (V3), Hu3A5 (V4), Hu3A5 (V5), Hu3A5 (V6), Hu3A5 (V7), Hu3A5 (V8), Hu3A5 (V9), Hu3A5 (V10), Hu3A5 (V11), Hu3A5 (V12), Hu3A5 (V13), Hu3A5 (V14), Hu3A5 (V15) and Hu3A5 (V16), respectively, and amino acid sequences were analyzed.

Sequence information on the heavy chain variable region and the light chain variable region of the antibody according to the sequencing results are shown in Tables 5 to 20 below. The underlined regions indicate complementarity determining regions (CDRs), and the CDRs of 16 humanized 3A5 antibodies are all identical to the CDRs of the mouse 3A5 antibody (Table 1).

In Tables 5 to 20, scFv refers to an antibody having a structure of a light chain variable region-linker-heavy chain variable region, and underlined portions in the scFv amino acid sequence and scFv nucleotide sequence indicate the linker portion.

**[Table 5]**

| Sequence information of Hu3A5 (V1) antibody | | |
|---|---|---|
| Hu3A5(V1) | sequence information | SEQ ID NO: |
| amino acid sequence of heavy chain variable region | EVQLVQSGAEVKKPGASVKVSCKASGYTFTSYWMHWMR QAPGQGLEWIGVIDPSDSYTSYNQKFQGRVTLTVDTSTSTA YMELSSLRSEDTAVYYCARGGKRAMDYWGQGTTVTVSS | SEQ ID NO: 11 |
| amino acid sequence of light chain variable region | DVVMTQSPLSLPVTLGQPASISCRSSQSLVHSNGNTYLHWF QQRPGQSPRLLIYKVSNRFSGVPDRFSGSGSGTDFTLKISRVE AEDVGVYFCSQSTHVPFTFGGGTKLEIK | SEQ ID NO: 12 |
| nucleotide sequence of heavy chain variable region | GAGGTGCAGCTGGTGCAGAGCGGCGCGGAGGTGAAGAA GCCTGGTGCTAGCGTGAAAGTTTCCTGTAAAGCGTCTGGC TACACGTTCACAAGCTATTGGATGCACTGGATGCGCCAG GCCCCCGGCCAGGGCTTGGAGTGGATCGGTGTGATTGAC CCGTCCGACAGCTACACCAGCTACAACCAGAAGTTCCAG GGTCGTGTCACCCTCACTGTGGACACCTCGACCTCCACCG CCTACATGGAGCTGAGCTCCCTACGGTCTGAAGATACAG CCGTGTACTACTGTGCTCGTGGTGGCAAGCGCGCCATGGA TTATTGGGGCCAGGGCACCACCGTCACTGTATCGTCC | SEQ ID NO: 13 |
| nucleotide sequence of light chain variable region | GATGTGGTGATGACCCAGTCTCCTCTTTCCCTGCCCGTAA CCCTCGGACAGCCAGCATCTATCTCATGCCGATCCTCCCA GAGCCTTGTCCACTCAAATGGCAACACGTACCTGCATTGG TTCCAGCAGAGGCCTGGGCAGAGCCCGCGCCTGCTGATC TACAAGGTGAGTAACCGCTTTTCTGGGGTGCCCGACCGCT TCAGTGGGTCGGGCTCCGGGACCGACTTTACTCTGAAGAT TTCCCGCGTGGAGGCCGAGGACGTGGGCGTCTACTTCTGC TCTCAATCTACTCACGTTCCCTTCACCTTCGGCGGCGGTA CCAAGCTGGAGATCAAG | SEQ ID NO: 14 |
| scFv amino acid sequence | DVVMTQSPLSLPVTLGQPASISCRSSQSLVHSNGNTYLHWF QQRPGQSPRLLIYKVSNRFSGVPDRFSGSGSGTDFTLKISRVE AEDVGVYFCSQSTHVPFTFGGGTKLEIKGGGGSGGGGSGG GGSEVQLVQSGAEVKKPGASVKVSCKASGYTFTSYWMHW MRQAPGQGLEWIGVIDPSDSYTSYNQKFQGRVTLTVDTSTS TAYMELSSLRSEDTAVYYCARGGKRAMDYWGQGTTVTVS S | SEQ ID NO: 15 |
| scFv nucleotide sequence | GATGTGGTGATGACCCAGTCTCCTCTTTCCCTGCCCGTAA CCCTCGGACAGCCAGCATCTATCTCATGCCGATCCTCCCA GAGCCTTGTCCACTCAAATGGCAACACGTACCTGCATTGG TTCCAGCAGAGGCCTGGGCAGAGCCCGCGCCTGCTGATC TACAAGGTGAGTAACCGCTTTTCTGGGGTGCCCGACCGCT TCAGTGGGTCGGGCTCCGGGACCGACTTTACTCTGAAGAT TTCCCGCGTGGAGGCCGAGGACGTGGGCGTCTACTTCTGC TCTCAATCTACTCACGTTCCCTTCACCTTCGGCGGCGGTA CCAAGCTGGAGATCAAGGGCGGCGGAGGCTCCGGTGG AGGCGGGTCCGGGGGAGGCGGCTCGGAGGTGCAGCTG GTGCAGAGCGGCGCGGAGGTGAAGAAGCCTGGTGCTAGC GTGAAAGTTTCCTGTAAAGCGTCTGGCTACACGTTCACAA GCTATTGGATGCACTGGATGCGCCAGGCCCCCGGCCAGG GCTTGGAGTGGATCGGTGTGATTGACCCGTCCGACAGCTA CACCAGCTACAACCAGAAGTTCCAGGGTCGTGTCACCCTC ACTGTGGACACCTCGACCTCCACCGCCTACATGGAGCTGA GCTCCCTACGGTCTGAAGATACAGCCGTGTACTACTGTGC TCGTGGTGGCAAGCGCGCCATGGATTATTGGGGCCAGGG CACCACCGTCACTGTATCGTCC | SEQ ID NO: 16 |

**[Table 6]**

| Sequence information of Hu3A5(V2) antibody | | |
|---|---|---|
| Hu3A5(V2) | sequence information | SEQ ID NO: |
| amino acid sequence of heavy chain variable region | EVQLVQSGAEVKKPGASVKVSCKASGYTFTSYWMHWM RQAPGQGLEWIGVIDPSDSYTSYNQKFQGRVTLTVDTSTS TAYMELSSLRSEDTAVYYCARGGKRAMDYWGQGTTVTV SS | SEQ ID NO: 17 |
| amino acid sequence of light chain variable region | DVVMTQSPLSLPVTLGQPASISCRSSQSLVHSNGNTYLHW FQQRPGQSPRLLIYKVSNRFSGVPDRFSGSGSGTDFTLKISR VEAEDVGVYYC SQSTHVPFT FGGGTKLEIK | SEQ ID NO: 18 |
| nucleotide sequence of heavy chain variable region | GAGGTGCAGCTGGTGCAGTCTGGTGCAGAGGTGAAGAA GCCTGGTGCTTCCGTGAAAGTATCTTGTAAGGCTTCTGG CTACACGTTCACCAGCTATTGGATGCACTGGATGCGCCA GGCCCCCGGCCAGGGTTTGGAGTGGATCGGTGTGATTG ACCCGTCCGACAGCTACACCTCCTACAACCAGAAATTTC AGGGCCGCGTCACTCTTACCGTGGATACCAGCACTTCGA CCGCCTACATGGAGCTGTCCTCTCTGCGCAGCGAGGACA CCGCGGTGTACTACTGCGCCCGTGGCGGCAAGCGCGCC ATGGATTATTGGGGCCAGGGCACTACCGTCACAGTGTCC AGC | SEQ ID NO: 19 |
| nucleotide sequence of light chain variable region | GACGTGGTGATGACCCAGAGTCCACTTTCGCTGCCTGTC ACACTAGGACAGCCGGCGTCCATCTCGTGCCGATCGTCC CAAAGCCTCGTCCACTCAAATGGCAACACGTACCTGCAT TGGTTCCAGCAGCGCCCCGGGCAGAGCCCACGTCTCCTG ATCTACAAGGTGAGTAACCGCTTCTCTGGCGTTCCCGAC AGGTTTTCAGGATCTGGGTCCGGCACCGACTTCACCCTG AAGATCTCTCGGGTGGAGGCTGAAGATGTGGGCGTTTA TTACTGTTCGCAGAGCACTCACGTCCCCTTCACCTTCGG CGGGGGGACCAAGCTGGAGATCAAG | SEQ ID NO: 20 |
| scFv amino acid sequence | DVVMTQSPLSLPVTLGQPASISCRSSQSLVHSNGNTYLHWF QQRPGQSPRLLIYKVSNRFSGVPDRFSGSGSGTDFTLKISRV EAEDVGVYYCSQSTHVPFTFGGGTKLEIKGGGGSGGGGS GGGGSEVQLVQSGAEVKKPGASVKVSCKASGYTFTSYW MHWMRQAPGQGLEWIGVIDPSDSYTSYNQKFQGRVTLTV DTSTSTAYMELSSLRSEDTAVYYCARGGKRAMDYWGQG TTVTVSS | SEQ ID NO: 21 |
| scFv nucleotide sequence | GACGTGGTGATGACCCAGAGTCCACTTTCGCTGCCTGTC ACACTAGGACAGCCGGCGTCCATCTCGTGCCGATCGTCC CAAAGCCTCGTCCACTCAAATGGCAACACGTACCTGCAT TGGTTCCAGCAGCGCCCCGGGCAGAGCCCACGTCTCCTG ATCTACAAGGTGAGTAACCGCTTCTCTGGCGTTCCCGAC AGGTTTTCAGGATCTGGGTCCGGCACCGACTTCACCCTG AAGATCTCTCGGGTGGAGGCTGAAGATGTGGGCGTTTA TTACTGTTCGCAGAGCACTCACGTCCCCTTCACCTTCGG CGGGGGGACCAAGCTGGAGATCAAGGGCGGGGGAGG CTCCGGCGGAGGCGGCTCCGGTGGTGGTGGCTCC GAGGTGCAGCTGGTGCAGTCTGGTGCAGAGGTGAAGAA GCCTGGTGCTTCCGTGAAAGTATCTTGTAAGGCTTCTGG CTACACGTTCACCAGCTATTGGATGCACTGGATGCGCCA GGCCCCCGGCCAGGGTTTGGAGTGGATCGGTGTGATTG ACCCGTCCGACAGCTACACCTCCTACAACCAGAAATTTC AGGGCCGCGTCACTCTTACCGTGGATACCAGCACTTCGA CCGCCTACATGGAGCTGTCCTCTCTGCGCAGCGAGGACA CCGCGGTGTACTACTGCGCCCGTGGCGGCAAGCGCGCC ATGGATTATTGGGGCCAGGGCACTACCGTCACAGTGTCC AGC | SEQ ID NO: 22 |

**[Table 7]**

| Sequence information of Hu3A5(V3) antibody | | |
|---|---|---|
| Hu3A5(V3) | sequence information | SEQ ID NO: |
| amino acid sequence of heavy chain variable region | EVQLVQSGAEVKKPGASVKVSCKASGYTFTSYWMHW MRQAPGQGLEWIGVIDPSDSYTSYNQKFQGRVTLTVDTS TSTAYMELSSLRSEDTAVYYCARGGKRAMDYWGQGTT VTVSS | SEQ ID NO: 23 |
| amino acid sequence of light chain variable region | DVVMTQSPDSLAVSLGERATINCKSSQSLVHSNGNTYLH WYQQKPGQPPKLLIYKVSNRFSGVPDRFSGSGSGTDFTL TISSLQAEDVAVYFC SQSTHVPFT FGGGTKLEIK | SEQ ID NO: 24 |
| nucleotide sequence of heavy chain variable region | GATGTGGTGATGACCCAGAGTCCCGACAGCCTGGCGG TGTCTCTTGGGGAGCGGGCGACTATTAACTGCAAAAG CTCCCAGAGCCTAGTCCACTCAAATGGCAACACTTACC TGCACTGGTACCAGCAGAAGCCTGGTCAGCCACCCAA GCTGCTCATCTACAAGGTTAGCAACAGGTTTTCAGGCG TCCCTGACCGCTTCAGTGGCTCCGGCTCCGGCACCGAC TTCACCCTGACCATCTCTTCTTTGCAGGCCGAGGACGT GGCGGTTTATTTCTGTTCTCAATCCACCCACGTCCCCTT CACCTTCGGCGGCGGGACCAAGCTGGAGATCAAA | SEQ ID NO: 25 |
| nucleotide sequence of light chain variable region | GAGGTGCAGCTGGTGCAGTCCGGGGCAGAGGTAAAGA AGCCGGGCGCTTCCGTGAAGGTGTCGTGTAAGGCTTCT GGCTACACGTTCACATCCTACTGGATGCATTGGATGCG CCAGGCTCCCGGACAGGGCCTGGAGTGGATCGGTGTG ATTGACCCGAGCGATTCTTACACCAGCTACAACCAGA AATTTCAGGGACGAGTGACTCTTACGGTGGACACCTC GACCTCCACTGCCTACATGGAGCTGAGCTCTCTGCGCT CGGAAGACACCGCCGTGTACTACTGCGCCCGTGGTGG CAAGCGCGCCATGGATTATTGGGGCCAGGGTACCACA GTCACCGTGTCGTCC | SEQ ID NO: 26 |
| scFv amino acid sequence | DVVMTQSPDSLAVSLGERATINCKSSQSLVHSNGNTYLH WYQQKPGQPPKLLIYKVSNRFSGVPDRFSGSGSGTDFTL TISSLQAEDVAVYFCSQSTHVPFTFGGGTKLEIKGGGGS GGGGSGGGGSEVQLVQSGAEVKKPGASVKVSCKASGY TFTSYWMHWMRQAPGQGLEWIGVIDPSDSYTSYNQKFQ GRVTLTVDTSTSTAYMELSSLRSEDTAVYYCARGGKRA MDYWGQGTTVTVSS | SEQ ID NO: 27 |
| scFv nucleotide sequence | GATGTGGTGATGACCCAGAGTCCCGACAGCCTGGCGG TGTCTCTTGGGGAGCGGGCGACTATTAACTGCAAAAG CTCCCAGAGCCTAGTCCACTCAAATGGCAACACTTACC TGCACTGGTACCAGCAGAAGCCTGGTCAGCCACCCAA GCTGCTCATCTACAAGGTTAGCAACAGGTTTTCAGGCG TCCCTGACCGCTTCAGTGGCTCCGGCTCCGGCACCGAC TTCACCCTGACCATCTCTTCTTTGCAGGCCGAGGACGT GGCGGTTTATTTCTGTTCTCAATCCACCCACGTCCCCTT CACCTTCGGCGGCGGGACCAAGCTGGAGATCAAAGGC GGAGGCGGCTCCGGTGGGGGGGGCTCGGGCGGCG GTGGCTCCGAGGTGCAGCTGGTGCAGTCCGGGGCAG AGGTAAAGAAGCCGGGCGCTTCCGTGAAGGTGTCGTG TAAGGCTTCTGGCTACACGTTCACATCCTACTGGATGC ATTGGATGCGCCAGGCTCCCGGACAGGGCCTGGAGTG GATCGGTGTGATTGACCCGAGCGATTCTTACACCAGCT ACAACCAGAAATTTCAGGGACGAGTGACTCTTACGGT GGACACCTCGACCTCCACTGCCTACATGGAGCTGAGCT CTCTGCGCTCGGAAGACACCGCCGTGTACTACTGCGCC CGTGGTGGCAAGCGCGCCATGGATTATTGGGGCCAGG GTACCACAGTCACCGTGTCGTCC | SEQ ID NO: 28 |

**[Table 8]**

| Sequence information of Hu3A5 (V4) antibody | | |
|---|---|---|
| Hu3A5 (V4) | sequence information | SEQ ID NO: |
| amino acid sequence of heavy chain variable region | EVQLVQSGAEVKKPGASVKVSCKASGYTFTSYWMH WMRQAPGQGLEWIGVIDPSDSYTSYNQKFQGRVTLT VDTSTSTAYMELSSLRSEDTAVYYCARGGKRAMDY WGQGTTVTVSS | SEQ ID NO: 29 |
| amino acid sequence of light chain variable region | DVVMTQSPDSLAVSLGERATINCKSSQSLVHSNGNT YLHWYQQKPGQPPKLLIYKVSNRFSGVPDRFSGSGSG TDFTLTISSLQAEDVAVYYCSQSTHVPFTFGGGTKLEI K | SEQ ID NO: 30 |
| nucleotide sequence of heavy chain variable region | GATGTGGTGATGACCCAGTCCCCCGACTCGCTCGC GGTGTCCCTGGGCGAGCGGGCTACCATTAACTGCA AGTCCTCCCAGTCTTTGGTCCACTCTAATGGCAACA CGTACCTGCACTGGTACCAGCAGAAGCCCGGCCAG CCGCCTAAACTGCTTATCTACAAGGTGTCTAACCGC TTCTCTGGCGTCCCAGACAGGTTTTCAGGCAGTGGG TCCGGTACCGACTTTACTCTGACCATCTCCTCGCTG CAGGCTGAGGACGTGGCGGTATATTACTGTTCGCA GAGCACACATGTTCCCTTCACCTTCGGCGGGGGCA CCAAGTTGGAGATCAAG | SEQ ID NO: 31 |
| nucleotide sequence of light chain variable region | GAGGTGCAGCTGGTGCAAAGTGGGGCAGAGGTGA AGAAGCCTGGCGCTTCCGTGAAAGTCTCATGCAAG GCCTCTGGCTACACGTTCACCTCTTATTGGATGCAC TGGATGCGCCAGGCCCCCGGACAGGGACTAGAGTG GATCGGTGTCATTGACCCGAGCGATAGCTACACGA GCTACAACCAGAAATTCCAGGGCCGAGTGACTCTC ACTGTGGACACCTCGACCTCCACCGCCTACATGGA GCTGAGCTCCCTGCGCAGCGAAGACACAGCGGTGT ACTACTGTGCCCGTGGGGGCAAGCGCGCCATGGAT TATTGGGGCCAGGGTACTACCGTGACCGTTTCGAG C | SEQ ID NO: 32 |
| scFv amino acid sequence | DVVMTQSPDSLAVSLGERATINCKSSQSLVHSNGNTY LHWYQQKPGQPPKLLIYKVSNRFSGVPDRFSGSGSGT DFTLTISSLQAEDVAVYYCSQSTHVPFTFGGGTKLEIK GGGGSGGGGSGGGGSEVQLVQSGAEVKKPGASVK VSCKASGYTFTSYWMHWMRQAPGQGLEWIGVIDPS DSYTSYNQKFQGRVTLTVDTSTSTAYMELSSLRSEDT AVYYCARGGKRAMDYWGQGTTVTVSS | SEQ ID NO: 33 |
| scFv nucleotide sequence | GATGTGGTGATGACCCAGTCCCCCGACTCGCTCGC GGTGTCCCTGGGCGAGCGGGCTACCATTAACTGCA AGTCCTCCCAGTCTTTGGTCCACTCTAATGGCAACA CGTACCTGCACTGGTACCAGCAGAAGCCCGGCCAG CCGCCTAAACTGCTTATCTACAAGGTGTCTAACCGC TTCTCTGGCGTCCCAGACAGGTTTTCAGGCAGTGGG TCCGGTACCGACTTTACTCTGACCATCTCCTCGCTG CAGGCTGAGGACGTGGCGGTATATTACTGTTCGCA GAGCACACATGTTCCCTTCACCTTCGGCGGGGGCA CCAAGTTGGAGATCAAGGGTGGCGGAGGTTCCGGG GGGGGCGGCTCTGGTGGCGGCGGCTCCGAGGT GCAGCTGGTGCAAAGTGGGGCAGAGGTGAAGAAG CCTGGCGCTTCCGTGAAAGTCTCATGCAAGGCCTCT GGCTACACGTTCACCTCTTATTGGATGCACTGGATG CGCCAGGCCCCCGGACAGGGACTAGAGTGGATCGG TGTCATTGACCCGAGCGATAGCTACACGAGCTACA ACCAGAAATTCCAGGGCCGAGTGACTCTCACTGTG GACACCTCGACCTCCACCGCCTACATGGAGCTGAG CTCCCTGCGCAGCGAAGACACAGCGGTGTACTACT GTGCCCGTGGGGGCAAGCGCGCCATGGATTATTGG GGCCAGGGTACTACCGTGACCGTTTCGAGC | SEQ ID NO: 34 |

**[Table 9]**

| Sequence information of Hu3A5 (V5) antibody | | |
|---|---|---|
| Hu 3A5 (V5) | sequence information | SEQ ID NO: |
| amino acid sequence of heavy chain variable region | QVQLVQSGAEVKKPGASVKVSCKAS GYTFTSYW MHWVRQAPGQGLEWMGV IDPSDSYT SYNQKFQGRVTMTVDTSTSTAYMELSSLRSEDTAVY YC ARGGKRAMDY WGQGTTVTVSS | SEQ ID NO: 35 |
| amino acid sequence of light chain variable region | DVVMTQSPLSLPVTLGQPASISCRSS QSLVHSNGNTY LHWFQQRPGQSPRLLIY KVS NRFSGVPDRFSGSGSGTDFTLKISRVEAEDVGVYFC SQSTHVPFT FGGGTKLEIK | SEQ ID NO: 36 |
| nucleotide sequence of heavy chain variable region | CAGGTCCAGCTGGTGCAGAGTGGAGCAGAGGTGAA GAAGCCTGGTGCGTCTGTTAAAGTTTCTTGCAAGGC GTCCGGCTACACGTTCACAAGCTATTGGATGCACTG GGTCCGCCAGGCCCCAGGGCAGGGCTTGGAGTGGA TGGGCGTGATTGACCCGTCCGACAGCTACACCAGC TACAACCAGAAATTTCAGGGACGTGTCACTATGAC AGTGGACACTTCGACCTCTACTGCCTACATGGAACT TTCCTCTCTACGCTCGGAGGACACCGCCGTGTACTA CTGTGCTCGTGGGGGCAAGCGCGCCATGGATTATT GGGGCCAGGGCACCACGGTCACCGTGTCCTCT | SEQ ID NO: 37 |
| nucleotide sequence of light chain variable region | GACGTGGTGATGACCCAGTCCCCCCTGAGCCTTCCA GTGACCCTCGGCCAGCCGGCTTCCATCTCATGCCGA AGCTCCCAGAGCCTCGTCCACTCAAATGGCAACAC GTACCTGCACTGGTTCCAGCAGCGGCCCGGGCAGA GCCCTCGCCTGCTGATCTACAAGGTGTCGAACCGCT TCTCGGGCGTCCCCGACAGGTTTTCGGGTTCCGGTA GTGGTACCGACTTCACCCTGAAGATCTCCCGCGTGG AGGCTGAGGATGTGGGCGTATACTTTTGTTCTCAAT CCACTCATGTGCCCTTCACCTTCGGAGGCGGCACCA AGCTGGAGATCAAG | SEQ ID NO: 38 |
| scFv amino acid sequence | DVVMTQSPLSLPVTLGQPASISCRSSQSLVHSNGNTYL HWFQQRPGQSPRLLIYKVSNRFSGVPDRFSGSGSGTD FTLKISRVEAEDVGVYFCSQSTHVPFTFGGGTKLEIKG GGGSGGGGSGGGGSQVQLVQSGAEVKKPGASVKV SCKASGYTFTSYWMHWVRQAPGQGLEWMGVIDPSD SYTSYNQKFQGRVTMTVDTSTSTAYMELSSLRSEDTA VYYCARGGKRAMDYWGQGTTVTVSS | SEQ ID NO: 39 |
| scFv nucleotide sequence | GACGTGGTGATGACCCAGTCCCCCCTGAGCCTTCCA GTGACCCTCGGCCAGCCGGCTTCCATCTCATGCCGA AGCTCCCAGAGCCTCGTCCACTCAAATGGCAACAC GTACCTGCACTGGTTCCAGCAGCGGCCCGGGCAGA GCCCTCGCCTGCTGATCTACAAGGTGTCGAACCGCT TCTCGGGCGTCCCCGACAGGTTTTCGGGTTCCGGTA GTGGTACCGACTTCACCCTGAAGATCTCCCGCGTGG AGGCTGAGGATGTGGGCGTATACTTTTGTTCTCAAT CCACTCATGTGCCCTTCACCTTCGGAGGCGGCACCA AGCTGGAGATCAAG GGCGGGGGCGGGTCTGGAGGTGGCGGCTCCGG TGGTGGGGGCTCC CAGGTCCAGCTGGTGCAGAGTGGAGCAGAGGTGAA GAAGCCTGGTGCGTCTGTTAAAGTTTCTTGCAAGGC GTCCGGCTACACGTTCACAAGCTATTGGATGCACTG GGTCCGCCAGGCCCCAGGGCAGGGCTTGGAGTGGA TGGGCGTGATTGACCCGTCCGACAGCTACACCAGC TACAACCAGAAATTTCAGGGACGTGTCACTATGAC AGTGGACACTTCGACCTCTACTGCCTACATGGAACT TTCCTCTCTACGCTCGGAGGACACCGCCGTGTACTA CTGTGCTCGTGGGGGCAAGCGCGCCATGGATTATT GGGGCCAGGGCACCACGGTCACCGTGTCCTCT | SEQ ID NO: 40 |

**[Table 10]**

| Sequence information of Hu3A5(V6) antibody | | |
|---|---|---|
| Hu 3A5(V6) | sequence information | sequence number |
| amino acid sequence of heavy chain variable region | QVQLVQSGAEVKKPGASVKVSCKAS GYTFTSYW MHWVRQAPGQGLEWMGV IDPSDSYT SYNQKFQGRVTMTVDTSTSTAYMELSSLRSEDTAV YYC ARGGKRAMDY WGQGTTVTVSS | SEQ ID NO: 41 |
| amino acid sequence of light chain variable region | DVVMTQSPLSLPVTLGQPASISCRSS QSLVHSNGNTY LHWFQQRPGQSPRLLIY KVS NRFSGVPDRFSGSGSGTDFTLKISRVEAEDVGVYYC SQSTHVPFT FGGGTKLEIK | SEQ ID NO: 42 |
| nucleotide sequence of heavy chain variable region | CAGGTGCAGCTAGTGCAGTCTGGGGCGGAGGTGA AGAAGCCTGGCGCTTCCGTGAAAGTTTCGTGTAA GGCATCCGGCTACACGTTCACCAGCTATTGGATGC ATTGGGTCCGCCAGGCCCCCGGCCAGGGTCTGGA GTGGATGGGCGTGATTGACCCGTCCGACAGTTAC ACCAGTTACAACCAGAAGTTCCAGGGCCGGGTGA CCATGACTGTGGACACCTCGACCTCCACTGCCTAC ATGGAGCTGAGCAGCCTGCGCTCGGAGGACACCG CGGTGTACTACTGCGCCCGTGGTGGGAAGCGCGC CATGGATTATTGGGGCCAGGGCACCACCGTGACT GTCAGCTCC | SEQ ID NO: 43 |
| nucleotide sequence of light chain variable region | GATGTGGTGATGACCCAGTCCCCTTTGTCCCTTCC CGTCACCTTGGGTCAGCCAGCTTCTATCTCTTGCC GAAGCTCTCAGTCTCTGGTCCACTCTAATGGCAAC ACATACCTGCACTGGTTCCAGCAGAGACCAGGTC AGTCGCCGCGCCTGCTGATCTACAAGGTGAGCAA CCGTTTCTCCGGAGTCCCCGACAGGTTTTCAGGCT CCGGGTCAGGGACCGACTTTACACTCAAAATCTC GCGCGTGGAGGCTGAAGATGTTGGCGTGTATTAC TGTTCTCAAAGCACTCACGTACCCTTCACCTTCGG CGGAGGCACCAAGCTGGAGATCAAG | SEQ ID NO: 44 |
| scFv amino acid sequence | DVVMTQSPLSLPVTLGQPASISCRSSQSLVHSNGNTY LHWFQQRPGQSPRLLIYKVSNRFSGVPDRFSGSGSG TDFTLKISRVEAEDVGVYYCSQSTHVPFTFGGGTKL EIKGGGGSGGGGSGGGGSQVQLVQSGAEVKKPGA SVKVSCKASGYTFTSYWMHWVRQAPGQGLEWMG VIDPSDSYTSYNQKFQGRVTMTVDTSTSTAYMELSS LRSEDTAVYYCARGGKRAMDYWGQGTTVTVSS | SEQ ID NO: 45 |
| scFv nucleotide sequence | GATGTGGTGATGACCCAGTCCCCTTTGTCCCTTCC CGTCACCTTGGGTCAGCCAGCTTCTATCTCTTGCC GAAGCTCTCAGTCTCTGGTCCACTCTAATGGCAAC ACATACCTGCACTGGTTCCAGCAGAGACCAGGTC AGTCGCCGCGCCTGCTGATCTACAAGGTGAGCAA CCGTTTCTCCGGAGTCCCCGACAGGTTTTCAGGCT CCGGGTCAGGGACCGACTTTACACTCAAAATCTC GCGCGTGGAGGCTGAAGATGTTGGCGTGTATTAC TGTTCTCAAAGCACTCACGTACCCTTCACCTTCGG CGGAGGCACCAAGCTGGAGATCAAG GGCGGGGGAGGCTCCGGTGGGGGCGGTAGCG GCGGCGGAGGCTCC CAGGTGCAGCTAGTGCAGTCTGGGGCGGAGGTGA AGAAGCCTGGCGCTTCCGTGAAAGTTTCGTGTAA GGCATCCGGCTACACGTTCACCAGCTATTGGATGC ATTGGGTCCGCCAGGCCCCCGGCCAGGGTCTGGA GTGGATGGGCGTGATTGACCCGTCCGACAGTTAC ACCAGTTACAACCAGAAGTTCCAGGGCCGGGTGA CCATGACTGTGGACACCTCGACCTCCACTGCCTAC ATGGAGCTGAGCAGCCTGCGCTCGGAGGACACCG CGGTGTACTACTGCGCCCGTGGTGGGAAGCGCGC CATGGATTATTGGGGCCAGGGCACCACCGTGACT GTCAGCTCC | SEQ ID NO: 46 |

**[Table 11]**

| Sequence information of Hu3A5 (V7) antibody | | |
|---|---|---|
| Hu 3A5 (V7) | sequence information | sequence number |
| amino acid sequence of heavy chain variable region | QVQLVQSGAEVKKPGASVKVSCKAS GYTFTSYW MHWVRQAPGQGLEWMGV IDPSDSYT SYNQKFQGRVTMTVDTSTSTAYMELSSLRSEDTA VYYC ARGGKRAMDY WGQGTTVTVSS | SEQ ID NO: 47 |
| amino acid sequence of light chain variable region | DVVMTQSPDSLAVSLGERATINCKSS QSLVHSNGNTY LHWYQQKPGQPPKLLIY KVS NRFSGVPDRFSGSGSGTDFTLTISSLQAEDVAVYFC SQSTHVPFT FGGGTKLEIK | SEQ ID NO: 48 |
| nucleotide sequence of heavy chain variable region | CAAGTGCAGCTGGTGCAGTCCGGCGCAGAGGTA AAGAAGCCCGGCGCTTCCGTGAAGGTGTCCTGT AAAGCGTCGGGCTACACGTTCACCAGCTATTGG ATGCACTGGGTGCGCCAGGCCCCCGGGCAGGGC CTGGAGTGGATGGGCGTGATTGACCCGAGTGAC AGCTACACCAGTTACAACCAGAAATTTCAGGGA CGTGTGACCATGACTGTGGACACCTCGACCTCTA CTGCCTACATGGAGCTGTCCTCTCTCCGCTCGGA GGACACCGCCGTGTACTACTGCGCCCGAGGCGG CAAGCGCGCCATGGATTATTGGGGCCAGGGCAC CACCGTCACCGTGTCGTCC | SEQ ID NO: 49 |
| nucleotide sequence of light chain variable region | GACGTGGTGATGACCCAGAGCCCAGATTCTCTG GCGGTATCTCTTGGCGAGCGGGCTACTATCAACT GCAAATCTTCCCAGAGCCTAGTCCACAGCAATG GGAACACGTACCTGCATTGGTACCAGCAGAAGC CTGGTCAGCCGCCTAAGCTGCTGATCTACAAGG TTAGCAACCGCTTCTCCGGTGTCCCCGACAGGTT TTCAGGCTCCGGCTCCGGGACCGACTTCACCCTG ACCATCTCGTCTTTGCAGGCTGAAGATGTGGCG GTCTACTTCTGTTCACAGAGCACACACGTTCCCT TCACCTTCGGCGGGGGCACTAAGCTGGAGATCA AG | SEQ ID NO: 50 |
| scFv amino acid sequence | DVVMTQSPDSLAVSLGERATINCKSSQSLVHSNGN TYLHWYQQKPGQPPKLLIYKVSNRFSGVPDRFSGS GSGTDFTLTISSLQAEDVAVYFCSQSTHVPFTFGGG TKLEIKGGGGSGGGGSGGGGSQVQLVQSGAEVK KPGASVKVSCKASGYTFTSYWMHWVRQAPGQGL EWMGVIDPSDSYTSYNQKFQGRVTMTVDTSTSTA YMELSSLRSEDTAVYYCARGGKRAMDYWGQGTT VTVSS | SEQ ID NO: 5 1 |
| scFv nucleotide sequence | GACGTGGTGATGACCCAGAGCCCAGATTCTCTG GCGGTATCTCTTGGCGAGCGGGCTACTATCAACT GCAAATCTTCCCAGAGCCTAGTCCACAGCAATG GGAACACGTACCTGCATTGGTACCAGCAGAAGC CTGGTCAGCCGCCTAAGCTGCTGATCTACAAGG TTAGCAACCGCTTCTCCGGTGTCCCCGACAGGTT | SEQ ID NO: 52 |
| | TTCAGGCTCCGGCTCCGGGACCGACTTCACCCTG ACCATCTCGTCTTTGCAGGCTGAAGATGTGGCG GTCTACTTCTGTTCACAGAGCACACACGTTCCCT TCACCTTCGGCGGGGGCACTAAGCTGGAGATCA AG GGCGGTGGTGGTTCCGGCGGAGGAGGCTCCG GAGGGGGTGGGTCT CAAGTGCAGCTGGTGCAGTCCGGCGCAGAGGTA AAGAAGCCCGGCGCTTCCGTGAAGGTGTCCTGT AAAGCGTCGGGCTACACGTTCACCAGCTATTGG ATGCACTGGGTGCGCCAGGCCCCCGGGCAGGGC CTGGAGTGGATGGGCGTGATTGACCCGAGTGAC AGCTACACCAGTTACAACCAGAAATTTCAGGGA CGTGTGACCATGACTGTGGACACCTCGACCTCTA CTGCCTACATGGAGCTGTCCTCTCTCCGCTCGGA GGACACCGCCGTGTACTACTGCGCCCGAGGCGG CAAGCGCGCCATGGATTATTGGGGCCAGGGCAC CACCGTCACCGTGTCGTCC | |

**[Table 12]**

| Sequence information of Hu3A5 (V8) antibody | | |
|---|---|---|
| Hu 3A5 (V8) | sequence information | SEQ ID NO: |
| amino acid sequence of heavy chain variable region | QVQLVQSGAEVKKPGASVKVSCKAS GYTFTSYW MHWVRQAPGQGLEWMGV IDPSDSYT SYNQKFQGRVTMTVDTSTSTAYMELSSLRSEDTAVYYC ARGGKRAMDY WGQGTTVTVSS | SEQ ID NO: 53 |
| amino acid sequence of light chain variable region | DVVMTQSPDSLAVSLGERATINCKSS QSLVHSNGNTY LHWYQQKPGQPPKLLIY KVS NRFSGVPDRFSGSGSGTDFTLTISSLQAEDVAVYYC SQSTHVPFT FGGGTKLEIK | SEQ ID NO: 5 4 |
| nucleotide sequence of heavy chain variable region | CAGGTCCAGCTGGTGCAGTCTGGCGCGGAGGTGAAGA AGCCAGGTGCTTCCGTGAAAGTGTCCTGTAAGGCCAG TGGCTACACGTTCACAAGCTATTGGATGCACTGGGTGC GCCAGGCACCCGGACAGGGTCTGGAATGGATGGGCGT GATTGACCCGTCCGACAGTTACACCAGCTACAACCAG AAATTTCAGGGACGTGTGACCATGACAGTTGACACCT CGACTTCCACTGCCTACATGGAGCTGTCCTCCCTCCGC TCGGAGGACACTGCCGTGTACTACTGTGCCCGAGGCG GCAAGCGCGCCATGGATTATTGGGGCCAGGGCACGAC CGTCACCGTGTCGTCA | SEQ ID NO: 55 |
| nucleotide sequence of light chain variable region | GATGTGGTGATGACCCAGAGCCCTGACTCTTTGGCGGT ATCGCTAGGCGAGCGGGCTACCATCAACTGCAAGTCC AGCCAGAGCCTTGTCCATTCTAATGGCAACACGTACCT GCACTGGTACCAACAGAAGCCCGGGCAGCCGCCTAAA CTGCTCATCTACAAGGTCAGCAACCGCTTCTCCGGCGT CCCCGACAGGTTTTCGGGCTCCGGCTCAGGGACCGACT TCACCCTGACCATCTCTTCTCTGCAGGCCGAGGATGTG GCGGTATACTACTGCTCTCAGAGCACTCACGTTCCCTT CACCTTCGGCGGAGGCACCAAGCTGGAGATCAAG | SEQ ID NO: 56 |
| scFv amino acid sequence | DVVMTQSPDSLAVSLGERATINCKSSQSLVHSNGNTYLH WYQQKPGQPPKLLIYKVSNRFSGVPDRFSGSGSGTDFTL TISSLQAEDVAVYYCSQSTHVPFTFGGGTKLEIKGGGGS GGGGSGGGGSQVQLVQSGAEVKKPGASVKVSCKASGY TFTSYWMHWVRQAPGQGLEWMGVIDPSDSYTSYNQKF QGRVTMTVDTSTSTAYMELSSLRSEDTAVYYCARGGKR AMDYWGQGTTVTVSS | SEQ ID NO: 57 |
| scFv nucleotide sequence | GATGTGGTGATGACCCAGAGCCCTGACTCTTTGGCGGT ATCGCTAGGCGAGCGGGCTACCATCAACTGCAAGTCC AGCCAGAGCCTTGTCCATTCTAATGGCAACACGTACCT GCACTGGTACCAACAGAAGCCCGGGCAGCCGCCTAAA CTGCTCATCTACAAGGTCAGCAACCGCTTCTCCGGCGT CCCCGACAGGTTTTCGGGCTCCGGCTCAGGGACCGACT TCACCCTGACCATCTCTTCTCTGCAGGCCGAGGATGTG GCGGTATACTACTGCTCTCAGAGCACTCACGTTCCCTT CACCTTCGGCGGAGGCACCAAGCTGGAGATCAAG GGCGGGGGTGGGTCTGGGGGTGGCGGTTCCGGAG GTGGAGGCTCC | SEQ ID NO: 58 |
| | CAGGTCCAGCTGGTGCAGTCTGGCGCGGAGGTGAAGA AGCCAGGTGCTTCCGTGAAAGTGTCCTGTAAGGCCAG TGGCTACACGTTCACAAGCTATTGGATGCACTGGGTGC GCCAGGCACCCGGACAGGGTCTGGAATGGATGGGCGT GATTGACCCGTCCGACAGTTACACCAGCTACAACCAG AAATTTCAGGGACGTGTGACCATGACAGTTGACACCT CGACTTCCACTGCCTACATGGAGCTGTCCTCCCTCCGC TCGGAGGACACTGCCGTGTACTACTGTGCCCGAGGCG GCAAGCGCGCCATGGATTATTGGGGCCAGGGCACGAC CGTCACCGTGTCGTCA | |

**[Table 13]**

| Sequence information of Hu3A5 (V9) antibody | | |
|---|---|---|
| Hu 3A5 (V9) | sequence information | SEQ ID NO: |
| amino acid sequence of heavy chain variable region | EVQLVQSGSELKKPGASVKVSCKAS GYTFTSYW MHWMRQAPGQGLEWIGV IDPSDSYT SYNQGFTGRFVLSVDTSVSTAYLQISSLKAEDTAVYY C ARGGKRAMDY WGQGTTVTVSS | SEQ ID NO: 59 |
| amino acid sequence of light chain variable region | DVVMTQSPLSLPVTLGQPASISCRSS QSLVHSNGNTY LHWFQQRPGQSPRLLIY KVS NRFSGVPDRFSGSGSGTDFTLKISRVEAEDVGVYFC SQSTHVPFT FGGGTKLEIK | SEQ ID NO: 60 |
| nucleotide sequence of heavy chain variable region | GAGGTGCAGCTGGTGCAGAGCGGTTCCGAGCTGAA GAAGCCCGGGGCTTCCGTGAAGGTGTCGTGTAAAG CTTCTGGCTACACGTTCACAAGCTATTGGATGCACT GGATGCGCCAGGCCCCTGGTCAGGGACTGGAGTGG ATCGGCGTAATTGACCCGTCCGACAGCTACACTAG CTACAACCAGGGCTTCACCGGCCGCTTCGTCCTTTC TGTTGATACCTCCGTGTCCACTGCCTACTTGCAAAT TTCTTCTCTGAAAGCGGAAGACACCGCCGTGTACTA CTGCGCTCGTGGTGGCAAGCGCGCCATGGATTATT GGGGCCAGGGCACCACCGTCACCGTGAGCAGC | SEQ ID NO: 61 |
| nucleotide sequence of light chain variable region | GATGTGGTGATGACCCAGTCTCCCCTGAGTCTACCT GTCACTCTTGGACAGCCAGCAAGCATCTCCTGCCG ATCCTCACAGTCCTTGGTACACTCGAATGGCAACAC GTACCTGCACTGGTTCCAGCAGAGACCGGGACAGA GCCCACGCCTGCTCATCTACAAGGTTAGTAACCGCT TTTCTGGCGTCCCCGACAGGTTTTCAGGTTCCGGCA GTGGAACCGACTTTACTCTGAAGATCTCCCGGGTG GAGGCGGAGGACGTGGGCGTGTACTTCTGTTCGCA GTCCACCCATGTCCCCTTCACCTTCGGCGGCGGCAC CAAGCTGGAGATCAAG | SEQ ID NO: 62 |
| scFv amino acid sequence | DVVMTQSPLSLPVTLGQPASISCRSSQSLVHSNGNTYL HWFQQRPGQSPRLLIYKVSNRFSGVPDRFSGSGSGTD FTLKISRVEAEDVGVYFCSQSTHVPFTFGGGTKLEIKG GGGSGGGGSGGGGSEVQLVQSGSELKKPGASVKVS CKASGYTFTSYWMHWMRQAPGQGLEWIGVIDPSDS YTSYNQGFTGRFVLSVDTSVSTAYLQISSLKAEDTAV YYCARGGKRAMDYWGQGTTVTVSS | SEQ ID NO: 63 |
| scFv nucleotide sequence | GATGTGGTGATGACCCAGTCTCCCCTGAGTCTACCT GTCACTCTTGGACAGCCAGCAAGCATCTCCTGCCG ATCCTCACAGTCCTTGGTACACTCGAATGGCAACAC GTACCTGCACTGGTTCCAGCAGAGACCGGGACAGA GCCCACGCCTGCTCATCTACAAGGTTAGTAACCGCT TTTCTGGCGTCCCCGACAGGTTTTCAGGTTCCGGCA GTGGAACCGACTTTACTCTGAAGATCTCCCGGGTG GAGGCGGAGGACGTGGGCGTGTACTTCTGTTCGCA GTCCACCCATGTCCCCTTCACCTTCGGCGGCGGCAC CAAGCTGGAGATCAAG GGCGGAGGCGGCTCGGGTGGGGGGGGCTCCGG CGGGGGTGGGTCG GAGGTGCAGCTGGTGCAGAGCGGTTCCGAGCTGAA GAAGCCCGGGGCTTCCGTGAAGGTGTCGTGTAAAG CTTCTGGCTACACGTTCACAAGCTATTGGATGCACT GGATGCGCCAGGCCCCTGGTCAGGGACTGGAGTGG ATCGGCGTAATTGACCCGTCCGACAGCTACACTAG CTACAACCAGGGCTTCACCGGCCGCTTCGTCCTTTC TGTTGATACCTCCGTGTCCACTGCCTACTTGCAAAT TTCTTCTCTGAAAGCGGAAGACACCGCCGTGTACTA CTGCGCTCGTGGTGGCAAGCGCGCCATGGATTATT GGGGCCAGGGCACCACCGTCACCGTGAGCAGC | SEQ ID NO: 64 |

**[Table 14]**

| Sequence information of Hu3A5 (V10) antibody | | |
|---|---|---|
| Hu 3A5 (V10) | sequence information | SEQ ID NO: |
| amino acid sequence of heavy chain variable region | EVQLVQSGSELKKPGASVKVSCKAS GYTFTSYW MHWMRQAPGQGLEWIGV IDPSDSYT SYNQGFTGRFVLSVDTSVSTAYLQISSLKAEDTAVYYC ARGGKRAMDY WGQGTTVTVSS | SEQ ID NO: 65 |
| amino acid sequence of light chain variable region | DVVMTQSPLSLPVTLGQPASISCRSS QSLVHSNGNTY LHWFQQRPGQSPRLLIY KVS NRFSGVPDRFSGSGSGTDFTLKISRVEAEDVGVYYC SQSTHVPFT FGGGTKLEIK | SEQ ID NO: 66 |
| nucleotide sequence of heavy chain variable region | GAGGTGCAGCTGGTGCAGAGCGGTTCGGAGCTGAAGA AGCCAGGTGCATCCGTGAAGGTATCATGCAAAGCTTC CGGCTACACGTTCACCAGCTATTGGATGCATTGGATGC GCCAGGCCCCTGGGCAGGGGCTTGAGTGGATCGGTGT GATTGACCCAAGTGACAGCTACACCAGCTACAACCAG GGCTTCACCGGCCGGTTCGTCCTCTCCGTTGATACCAG CGTATCCACGGCCTACCTGCAAATTTCCAGCCTGAAAG CGGAGGACACAGCTGTTTACTACTGTGCTCGTGGCGG GAAGCGCGCCATGGATTATTGGGGCCAGGGCACCACC GTCACCGTGTCCTCT | SEQ ID NO: 67 |
| nucleotide sequence of light chain variable region | GACGTGGTGATGACCCAGAGCCCCCTGTCGCTGCCGG TGACTCTTGGACAGCCGGCCTCCATCTCTTGCCGATCT TCCCAGTCCTTGGTCCACTCTAATGGCAACACGTACTT GCACTGGTTCCAGCAGCGTCCCGGGCAGAGCCCTCGC CTGCTGATCTACAAGGTGTCGAACCGCTTCTCGGGCGT CCCCGACAGGTTTTCAGGCTCCGGTAGTGGCACCGACT TTACTCTCAAGATCAGCCGCGTGGAGGCGGAAGATGT GGGCGTGTACTACTGTTCTCAGTCCACTCACGTCCCCT TCACCTTTGGCGGTGGGACCAAGCTAGAGATCAAG | SEQ ID NO: 68 |
| scFv amino acid sequence | DVVMTQSPLSLPVTLGQPASISCRSSQSLVHSNGNTYLH WFQQRPGQSPRLLIYKVSNRFSGVPDRFSGSGSGTDFTLK ISRVEAEDVGVYYCSQSTHVPFTFGGGTKLEIKGGGGSG GGGSGGGGSEVQLVQSGSELKKPGASVKVSCKASGYTF TSYWMHWMRQAPGQGLEWIGVIDPSDSYTSYNQGFTGR FVLSVDTSVSTAYLQISSLKAEDTAVYYCARGGKRAMD YWGQGTTVTVSS | SEQ ID NO: 69 |
| scFv nucleotide sequence | GACGTGGTGATGACCCAGAGCCCCCTGTCGCTGCCGG TGACTCTTGGACAGCCGGCCTCCATCTCTTGCCGATCT TCCCAGTCCTTGGTCCACTCTAATGGCAACACGTACTT GCACTGGTTCCAGCAGCGTCCCGGGCAGAGCCCTCGC CTGCTGATCTACAAGGTGTCGAACCGCTTCTCGGGCGT CCCCGACAGGTTTTCAGGCTCCGGTAGTGGCACCGACT TTACTCTCAAGATCAGCCGCGTGGAGGCGGAAGATGT GGGCGTGTACTACTGTTCTCAGTCCACTCACGTCCCCT TCACCTTTGGCGGTGGGACCAAGCTAGAGATCAAGGG CGGAGGCGGGTCTGGTGGCGGAGGTTCTGGAGGG GGCGGCTCCGAGGTGCAGCTGGTGCAGAGCGGTTCG GAGCTGAAGAAGCCAGGTGCATCCGTGAAGGTATCAT GCAAAGCTTCCGGCTACACGTTCACCAGCTATTGGATG CATTGGATGCGCCAGGCCCCTGGGCAGGGGCTTGAGT GGATCGGTGTGATTGACCCAAGTGACAGCTACACCAG CTACAACCAGGGCTTCACCGGCCGGTTCGTCCTCTCCG TTGATACCAGCGTATCCACGGCCTACCTGCAAATTTCC AGCCTGAAAGCGGAGGACACAGCTGTTTACTACTGTG CTCGTGGCGGGAAGCGCGCCATGGATTATTGGGGCCA GGGCACCACCGTCACCGTGTCCTCT | SEQ ID NO: 70 |

**[Table 15]**

| Sequence information of Hu3A5(V11) antibody | | |
|---|---|---|
| Hu3A5(V1 1) | sequence information | SEQ ID NO: |
| amino acid sequence of heavy chain variable region | EVQLVQSGSELKKPGASVKVSCKAS GYTFTSYW MHWMRQAPGQGLEWIGV IDPSDSYT SYNQGFTGRFVLSVDTSVSTAYLQISSLKAEDTAVYYC ARGGKRAMDY WGQGTTVTVSS | SEQ ID NO: 71 |
| amino acid sequence of light chain variable region | DVVMTQSPDSLAVSLGERATINCKSS QSLVHSNGNTY LHWYQQKPGQPPKLLIY KVS NRFSGVPDRFSGSGSGTDFTLTISSLQAEDVAVYFC SQSTHVPFT FGGGTKLEIK | SEQ ID NO: 72 |
| nucleotide sequence of heavy chain variable region | GAGGTGCAGCTGGTGCAGAGCGGATCGGAGCTGAAG AAGCCCGGGGCTTCCGTGAAGGTGTCGTGTAAGGCT TCTGGCTACACGTTTACAAGCTATTGGATGCATTGGA TGCGCCAGGCGCCGGGTCAGGGTCTGGAGTGGATCG GTGTGATTGACCCGAGCGATTCTTACACCAGCTACAA CCAGGGCTTCACCGGCCGCTTCGTCCTGTCCGTGGAC ACCTCCGTGTCCACCGCCTACCTGCAAATTTCCTCTC TTAAAGCGGAAGACACTGCCGTGTACTACTGCGCCC GCGGGGGCAAGCGCGCCATGGATTATTGGGGCCAGG GGACTACCGTGACTGTATCTTCC | SEQ ID NO: 73 |
| nucleotide sequence of light chain variable region | GATGTGGTGATGACCCAGAGTCCCGACAGCCTAGCA GTTTCCCTGGGGGAGCGGGCTACCATCAACTGCAAA AGCTCCCAGAGCCTTGTCCACAGCAACGGCAACACG TACTTGCACTGGTACCAGCAGAAGCCTGGACAGCCA CCCAAACTGCTCATCTACAAGGTTTCTAATCGTTTTT CAGGCGTCCCTGACAGGTTTTCAGGCTCCGGTAGTGG CACCGACTTCACCCTGACCATCTCTTCTCTGCAGGCC GAGGACGTGGCGGTGTATTTCTGTTCGCAGTCCACAC ACGTCCCCTTCACCTTCGGCGGGGGCACCAAGCTGG AGATCAAG | SEQ ID NO: 74 |
| scFv amino acid sequence | DVVMTQSPDSLAVSLGERATINCKSSQSLVHSNGNTYL HWYQQKPGQPPKLLIYKVSNRFSGVPDRFSGSGSGTDF TLTISSLQAEDVAVYFCSQSTHVPFTFGGGTKLEIKGGG GSGGGGSGGGGSEVQLVQSGSELKKPGASVKVSCKA SGYTFTSYWMHWMRQAPGQGLEWIGVIDPSDSYTSYN QGFTGRFVLSVDTSVSTAYLQISSLKAEDTAVYYCARG GKRAMDYWGQGTTVTVSS | SEQ ID NO: 75 |
| scFv nucleotide sequence | GATGTGGTGATGACCCAGAGTCCCGACAGCCTAGCA GTTTCCCTGGGGGAGCGGGCTACCATCAACTGCAAA AGCTCCCAGAGCCTTGTCCACAGCAACGGCAACACG TACTTGCACTGGTACCAGCAGAAGCCTGGACAGCCA CCCAAACTGCTCATCTACAAGGTTTCTAATCGTTTTT CAGGCGTCCCTGACAGGTTTTCAGGCTCCGGTAGTGG CACCGACTTCACCCTGACCATCTCTTCTCTGCAGGCC GAGGACGTGGCGGTGTATTTCTGTTCGCAGTCCACAC ACGTCCCCTTCACCTTCGGCGGGGGCACCAAGCTGG AGATCAAG GGCGGCGGAGGCTCCGGAGGGGGAGGCTCGGGT GGTGGCGGCTCC GAGGTGCAGCTGGTGCAGAGCGGATCGGAGCTGAAG AAGCCCGGGGCTTCCGTGAAGGTGTCGTGTAAGGCT TCTGGCTACACGTTTACAAGCTATTGGATGCATTGGA TGCGCCAGGCGCCGGGTCAGGGTCTGGAGTGGATCG GTGTGATTGACCCGAGCGATTCTTACACCAGCTACAA CCAGGGCTTCACCGGCCGCTTCGTCCTGTCCGTGGAC ACCTCCGTGTCCACCGCCTACCTGCAAATTTCCTCTC TTAAAGCGGAAGACACTGCCGTGTACTACTGCGCCC GCGGGGGCAAGCGCGCCATGGATTATTGGGGCCAGG GGACTACCGTGACTGTATCTTCC | SEQ ID NO: 76 |

**[Table 16]**

| Sequence information of Hu3A5 (V12) antibody | | |
|---|---|---|
| Hu3A 5 (V12) | sequence information | SEQ ID NO: |
| amino acid sequence of heavy chain variable region | EVQLVQSGSELKKPGASVKVSCKASGYTFTSYWMHWMR QAPGQGLEWIGVIDPSDSYTSYNQGFTGRFVLSVDTSVST AYLQISSLKAEDTAVYYCARGGKRAMDYWGQGTTVTVS S | SEQ ID NO: 77 |
| amino acid sequence of light chain variable region | DVVMTQSPDSLAVSLGERATINCKSSQSLVHSNGNTYLH WYQQKPGQPPKLLIYKVSNRFSGVPDRFSGSGSGTDFTLTI SSLQAEDVAVYYC SQSTHVPFT FGGGTKLEIK | SEQ ID NO: 78 |
| nucleotide sequence of heavy chain variable region | GAGGTGCAGCTGGTGCAGAGCGGCTCGGAGCTGAAGAA GCCCGGGGCTTCCGTGAAGGTCTCATGCAAAGCGTCTG GCTACACGTTCACTAGCTATTGGATGCACTGGATGCGCC AGGCTCCTGGGCAGGGTCTGGAGTGGATCGGTGTCATT GACCCGTCCGACAGTTACACCAGCTACAACCAGGGCTT CACCGGCCGCTTCGTCTTGTCCGTAGACACCTCCGTGTC CACCGCCTACCTGCAGATCTCTTCTCTTAAAGCGGAAGA TACTGCAGTGTACTACTGTGCTCGTGGTGGCAAGCGCGC CATGGATTATTGGGGCCAGGGGACCACAGTCACCGTGT CCTCC | SEQ ID NO: 79 |
| nucleotide sequence of light chain variable region | GATGTGGTGATGACCCAGAGCCCGGACAGCCTGGCCGT GTCTCTGGGCGAGCGGGCCACTATTAACTGCAAATCCTC TCAATCCCTAGTCCACTCAAATGGCAACACGTACCTCCA TTGGTACCAGCAGAAGCCTGGACAGCCACCCAAGCTGC TGATCTACAAGGTGAGCAACCGCTTCTCTGGTGTTCCCG ACAGGTTTTCAGGCTCCGGCTCGGGCACCGACTTTACCC TGACCATCTCGTCCTTGCAGGCCGAGGACGTGGCGGTGT ATTACTGTTCTCAGAGCACTCACGTTCCCTTCACCTTCG GCGGAGGCACCAAGCTGGAGATCAAG | SEQ ID NO: 80 |
| scFv amino acid sequence | DVVMTQSPDSLAVSLGERATINCKSSQSLVHSNGNTYLHW YQQKPGQPPKLLIYKVSNRFSGVPDRFSGSGSGTDFTLTISS LQAEDVAVYYCSQSTHVPFTFGGGTKLEIKGGGGSGGGG SGGGGSEVQLVQSGSELKKPGASVKVSCKASGYTFTSYW MHWMRQAPGQGLEWIGVIDPSDSYTSYNQGFTGRFVLSV DTSVSTAYLQISSLKAEDTAVYYCARGGKRAMDYWGQGT TVTVSS | SEQ ID NO: 81 |
| scFv nucleotide sequence | GATGTGGTGATGACCCAGAGCCCGGACAGCCTGGCCGT GTCTCTGGGCGAGCGGGCCACTATTAACTGCAAATCCTC TCAATCCCTAGTCCACTCAAATGGCAACACGTACCTCCA TTGGTACCAGCAGAAGCCTGGACAGCCACCCAAGCTGC TGATCTACAAGGTGAGCAACCGCTTCTCTGGTGTTCCCG ACAGGTTTTCAGGCTCCGGCTCGGGCACCGACTTTACCC TGACCATCTCGTCCTTGCAGGCCGAGGACGTGGCGGTGT ATTACTGTTCTCAGAGCACTCACGTTCCCTTCACCTTCG GCGGAGGCACCAAGCTGGAGATCAAG GGCGGGGGCGGCTCCGGTGGTGGAGGTAGTGGGGG AGGGGGCTCC GAGGTGCAGCTGGTGCAGAGCGGCTCGGAGCTGAAGAA GCCCGGGGCTTCCGTGAAGGTCTCATGCAAAGCGTCTG GCTACACGTTCACTAGCTATTGGATGCACTGGATGCGCC AGGCTCCTGGGCAGGGTCTGGAGTGGATCGGTGTCATT GACCCGTCCGACAGTTACACCAGCTACAACCAGGGCTT CACCGGCCGCTTCGTCTTGTCCGTAGACACCTCCGTGTC CACCGCCTACCTGCAGATCTCTTCTCTTAAAGCGGAAGA TACTGCAGTGTACTACTGTGCTCGTGGTGGCAAGCGCGC CATGGATTATTGGGGCCAGGGGACCACAGTCACCGTGT CCTCC | SEQ ID NO: 82 |

**[Table 17]**

| Sequence information of Hu3A5 (V13) antibody | | |
|---|---|---|
| Hu3A5 (V13) | sequence information | SEQ ID NO: |
| amino acid sequence of heavy chain variable region | QVQLVQSGSELKKPGASVKVSCKASGYTFTSYWMNWVR QAPGQGLEWMGVIDPSDSYTSYNQGFTGRFVFSVDTSVST AYLQISSLKAEDTAVYYCARGGKRAMDYWGQGTTVTVS S | SEQ ID NO: 83 |
| amino acid sequence of light chain variable region | DVVMTQSPLSLPVTLGQPASISCRSSQSLVHSNGNTYLHW FQQRPGQSPRLLIYKVSNRFSGVPDRFSGSGSGTDFTLKISR VEAEDVGVYFC SQSTHVPFT FGGGTKLEIK | SEQ ID NO: 84 |
| nucleotide sequence of heavy chain variable region | CAGGTGCAGCTGGTGCAGAGCGGGAGCGAGCTGAAGAA GCCCGGGGCTTCGGTGAAGGTTTCTTGTAAGGCGTCCGG CTACACGTTCACTAGCTACTGGATGAACTGGGTCCGCCA GGCCCCTGGTCAGGGGCTGGAGTGGATGGGCGTGATTG ACCCGTCCGACTCCTACACCTCCTACAACCAGGGCTTCA CCGGCCGCTTCGTCTTTTCTGTGGACACCTCCGTTTCCAC CGCCTACCTGCAAATTTCCTCGCTGAAAGCGGAGGACA CTGCTGTATACTACTGTGCACGGGGGGGCAAACGTGCC ATGGATTATTGGGGCCAGGGCACAACCGTGACTGTCTCC TCC | SEQ ID NO: 85 |
| nucleotide sequence of light chain variable region | GATGTGGTGATGACCCAGAGTCCACTCTCTCTGCCCGTG ACCCTTGGACAGCCGGCTAGCATCTCATGCCGATCTTCA CAGAGCTTGGTGCATTCAAATGGCAACACTTACCTACAC TGGTTCCAGCAGCGTCCAGGACAGAGCCCTCGCCTGCTG ATCTACAAGGTGAGCAACCGCTTTTCCGGTGTCCCCGAC AGGTTTAGTGGTTCGGGCTCCGGCACCGACTTCACCTTG AAGATCTCTCGCGTGGAGGCCGAAGATGTGGGGGTGTA TTTCTGCTCTCAGAGCACACACGTACCCTTCACCTTCGG CGGCGGCACCAAGCTGGAGATCAAG | SEQ ID NO: 86 |
| scFv amino acid sequence | DVVMTQSPLSLPVTLGQPASISCRSSQSLVHSNGNTYLHWF QQRPGQSPRLLIYKVSNRFSGVPDRFSGSGSGTDFTLKISRV EAEDVGVYFCSQSTHVPFTFGGGTKLEIKGGGGSGGGGS GGGGSQVQLVQSGSELKKPGASVKVSCKASGYTFTSYW MNWVRQAPGQGLEWMGVIDPSDSYTSYNQGFTGRFVFSV DTSVSTAYLQISSLKAEDTAVYYCARGGKRAMDYWGQGT TVTVSS | SEQ ID NO: 87 |
| scFv nucleotide sequence | GATGTGGTGATGACCCAGAGTCCACTCTCTCTGCCCGTG ACCCTTGGACAGCCGGCTAGCATCTCATGCCGATCTTCA CAGAGCTTGGTGCATTCAAATGGCAACACTTACCTACAC TGGTTCCAGCAGCGTCCAGGACAGAGCCCTCGCCTGCTG ATCTACAAGGTGAGCAACCGCTTTTCCGGTGTCCCCGAC AGGTTTAGTGGTTCGGGCTCCGGCACCGACTTCACCTTG AAGATCTCTCGCGTGGAGGCCGAAGATGTGGGGGTGTA TTTCTGCTCTCAGAGCACACACGTACCCTTCACCTTCGG CGGCGGCACCAAGCTGGAGATCAAGGGAGGTGGTGGG TCTGGCGGTGGCGGTTCGGGCGGAGGCGGCTCCCAG GTGCAGCTGGTGCAGAGCGGGAGCGAGCTGAAGAAGCC CGGGGCTTCGGTGAAGGTTTCTTGTAAGGCGTCCGGCTA CACGTTCACTAGCTACTGGATGAACTGGGTCCGCCAGGC CCCTGGTCAGGGGCTGGAGTGGATGGGCGTGATTGACC CGTCCGACTCCTACACCTCCTACAACCAGGGCTTCACCG GCCGCTTCGTCTTTTCTGTGGACACCTCCGTTTCCACCGC CTACCTGCAAATTTCCTCGCTGAAAGCGGAGGACACTGC TGTATACTACTGTGCACGGGGGGGCAAACGTGCCATGG ATTATTGGGGCCAGGGCACAACCGTGACTGTCTCCTCC | SEQ ID NO: 88 |

**[Table 181**

| Sequence information of Hu3A5 (V14) antibody | | |
|---|---|---|
| Hu3A 5 (V14) | sequence information | SEQ ID NO: |
| amino acid sequence of heavy chain variable region | QVQLVQSGSELKKPGASVKVSCKAS GYTFTSYW MNWVRQAPGQGLEWMGV IDPSDSYT SYNQGFTGRFVFSVDTSVSTAYLQISSLKAEDTAVYYC ARGGKRAMDY WGQGTTVTVSS | SEQ ID NO: 89 |
| amino acid sequence of light chain variable region | DVVMTQSPLSLPVTLGQPASISCRSS QSLVHSNGNTY LHWFQQRPGQSPRLLIY KVS NRFSGVPDRFSGSGSGTDFTLKISRVEAEDVGVYYC SQSTHVPFT FGGGTKLEIK | SEQ ID NO: 90 |
| nucleotide sequence of heavy chain variable region | CAGGTCCAGCTGGTGCAGTCTGGGTCCGAGCTGAAG AAGCCTGGCGCGTCCGTGAAGGTGTCGTGTAAAGCT TCCGGCTACACCTTCACCAGCTATTGGATGAACTGGG TGCGCCAGGCCCCCGGGCAGGGGCTGGAGTGGATGG GCGTGATTGACCCGTCCGATTCTTACACAAGCTACAA CCAGGGCTTCACCGGCCGGTTCGTGTTCTCCGTGGAC ACCTCCGTTAGCACTGCCTACCTGCAGATTTCGTCGC TCAAAGCAGAAGACACGGCCGTGTACTACTGTGCTC GCGGCGGCAAGCGCGCCATGGATTATTGGGGCCAGG GTACGACCGTCACCGTGTCCTCT | SEQ ID NO: 9 1 |
| nucleotide sequence of light chain variable region | GATGTGGTGATGACCCAGAGCCCCCTGTCCCTTCCAG TCACCCTGGGACAGCCAGCTTCCATCTCATGCCGAAG TTCTCAGAGTCTAGTCCATTCTAATGGCAACACTTAC CTCCACTGGTTCCAGCAGAGACCTGGGCAGTCCCCG CGCCTGCTTATCTACAAGGTCAGCAACCGCTTTTCAG GCGTACCCGACAGGTTTTCAGGATCGGGCAGCGGGA CCGACTTCACATTGAAGATCTCTCGTGTGGAGGCGG AGGACGTGGGCGTGTACTACTGCAGCCAATCGACTC ACGTTCCCTTCACCTTCGGCGGAGGCACCAAGCTGG AGATCAAG | SEQ ID NO: 92 |
| scFv amino acid sequence | DVVMTQSPLSLPVTLGQPASISCRSSQSLVHSNGNTYLH WFQQRPGQSPRLLIYKVSNRFSGVPDRFSGSGSGTDFTL KISRVEAEDVGVYYCSQSTHVPFTFGGGTKLEIKGGGG SGGGGSGGGGSQVQLVQSGSELKKPGASVKVSCKAS GYTFTSYWMNWVRQAPGQGLEWMGVIDPSDSYTSYN QGFTGRFVFSVDTSVSTAYLQISSLKAEDTAVYYCARG GKRAMDYWGQGTTVTVSS | SEQ ID NO: 93 |
| scFv nucleotide sequence | GATGTGGTGATGACCCAGAGCCCCCTGTCCCTTCCAG TCACCCTGGGACAGCCAGCTTCCATCTCATGCCGAAG TTCTCAGAGTCTAGTCCATTCTAATGGCAACACTTAC CTCCACTGGTTCCAGCAGAGACCTGGGCAGTCCCCG CGCCTGCTTATCTACAAGGTCAGCAACCGCTTTTCAG GCGTACCCGACAGGTTTTCAGGATCGGGCAGCGGGA CCGACTTCACATTGAAGATCTCTCGTGTGGAGGCGG AGGACGTGGGCGTGTACTACTGCAGCCAATCGACTC ACGTTCCCTTCACCTTCGGCGGAGGCACCAAGCTGG AGATCAAG GGCGGCGGCGGTTCTGGGGGCGGAGGTTCCGGT GGTGGTGGTTCC CAGGTCCAGCTGGTGCAGTCTGGGTCCGAGCTGAAG AAGCCTGGCGCGTCCGTGAAGGTGTCGTGTAAAGCT TCCGGCTACACCTTCACCAGCTATTGGATGAACTGGG TGCGCCAGGCCCCCGGGCAGGGGCTGGAGTGGATGG GCGTGATTGACCCGTCCGATTCTTACACAAGCTACAA CCAGGGCTTCACCGGCCGGTTCGTGTTCTCCGTGGAC ACCTCCGTTAGCACTGCCTACCTGCAGATTTCGTCGC TCAAAGCAGAAGACACGGCCGTGTACTACTGTGCTC GCGGCGGCAAGCGCGCCATGGATTATTGGGGCCAGG GTACGACCGTCACCGTGTCCTCT | SEQ ID NO: 94 |

**[Table 19]**

| Sequence information of Hu3A5 (V15) antibody | | |
|---|---|---|
| Hu3A 5 (V15) | sequence information | SEQ ID NO: |
| amino acid sequence of heavy chain variable region | QVQLVQSGSELKKPGASVKVSCKAS GYTFTSYW MNWVRQAPGQGLEWMGV IDPSDSYT SYNQGFTGRFVFSVDTSVSTAYLQISSLKAEDTAVY YC ARGGKRAMDY WGQGTTVTVSS | SEQ ID NO: 95 |
| amino acid sequence of light chain variable region | DVVMTQSPDSLAVSLGERATINCKSS QSLVHSNGNTY LHWYQQKPGQPPKLLIY KVS NRFSGVPDRFSGSGSGTDFTLTISSLQAEDVAVYFC SQSTHVPFT FGGGTKLEIK | SEQ ID NO: 96 |
| nucleotide sequence of heavy chain variable region | CAGGTGCAGCTGGTGCAGAGCGGCAGCGAGCTGA AGAAGCCTGGGGCTTCCGTAAAGGTCTCATGCAA GGCTTCGGGCTACACGTTCACAAGCTATTGGATG AACTGGGTGCGCCAGGCCCCTGGTCAGGGGCTGG AGTGGATGGGCGTCATTGACCCGAGTGACAGCTA CACCAGCTACAACCAGGGCTTCACCGGCCGGTTC GTCTTTTCCGTGGACACCTCCGTTTCCACTGCCTA CCTCCAAATTTCTTCTCTTAAAGCCGAGGACACTG CAGTGTACTACTGTGCGCGTGGTGGCAAGCGCGC CATGGATTACTGGGGCCAGGGTACTACCGTCACC GTGTCGTCC | SEQ ID NO: 97 |
| nucleotide sequence of light chain variable region | GATGTGGTGATGACCCAGTCCCCCGATTCTCTAGC CGTGTCCCTGGGCGAGCGCGCTACAATCAACTGC AAAAGCTCCCAGTCCTTGGTGCACAGCAATGGCA ACACGTACCTGCATTGGTACCAGCAGAAGCCCGG ACAGCCACCGAAGCTGCTCATCTACAAGGTGAGC AACCGCTTCAGTGGGGTCCCCGACAGGTTTTCAG GCTCCGGTTCGGGGACCGACTTTACTCTGACCATC TCCTCTCTGCAGGCGGAAGACGTGGCGGTATATTT CTGTTCTCAGTCCACCCACGTTCCCTTCACCTTCG GCGGAGGGACCAAGCTTGAGATCAAA | SEQ ID NO: 98 |
| scFv amino acid sequence | DVVMTQSPDSLAVSLGERATINCKSSQSLVHSNGNT YLHWYQQKPGQPPKLLIYKVSNRFSGVPDRFSGSGS GTDFTLTISSLQAEDVAVYFCSQSTHVPFTFGGGTKL EIKGGGGSGGGGSGGGGSQVQLVQSGSELKKPGA SVKVSCKASGYTFTSYWMNWVRQAPGQGLEWMG VIDPSDSYTSYNQGFTGRFVFSVDTSVSTAYLQISSL KAEDTAVYYCARGGKRAMDYWGQGTTVTVSS | SEQ ID NO: 99 |
| scFv nucleotide sequence | GATGTGGTGATGACCCAGTCCCCCGATTCTCTAGC CGTGTCCCTGGGCGAGCGCGCTACAATCAACTGC AAAAGCTCCCAGTCCTTGGTGCACAGCAATGGCA ACACGTACCTGCATTGGTACCAGCAGAAGCCCGG ACAGCCACCGAAGCTGCTCATCTACAAGGTGAGC AACCGCTTCAGTGGGGTCCCCGACAGGTTTTCAG GCTCCGGTTCGGGGACCGACTTTACTCTGACCATC TCCTCTCTGCAGGCGGAAGACGTGGCGGTATATTT CTGTTCTCAGTCCACCCACGTTCCCTTCACCTTCG GCGGAGGGACCAAGCTTGAGATCAAA GGCGGCGGCGGTTCTGGAGGTGGTGGGTCTGG CGGCGGAGGCTCC CAGGTGCAGCTGGTGCAGAGCGGCAGCGAGCTGA AGAAGCCTGGGGCTTCCGTAAAGGTCTCATGCAA GGCTTCGGGCTACACGTTCACAAGCTATTGGATG AACTGGGTGCGCCAGGCCCCTGGTCAGGGGCTGG AGTGGATGGGCGTCATTGACCCGAGTGACAGCTA CACCAGCTACAACCAGGGCTTCACCGGCCGGTTC GTCTTTTCCGTGGACACCTCCGTTTCCACTGCCTA CCTCCAAATTTCTTCTCTTAAAGCCGAGGACACTG CAGTGTACTACTGTGCGCGTGGTGGCAAGCGCGC CATGGATTACTGGGGCCAGGGTACTACCGTCACC GTGTCGTCC | SEQ ID NO: 100 |

**[Table 20]**

| Sequence information of Hu3A5 (V16) antibody | | |
|---|---|---|
| Hu3A 5 (V16) | sequence information | SEQ ID NO: |
| amino acid sequence of heavy chain variable region | QVQLVQSGSELKKPGASVKVSCKAS GYTFTSYW MNWVRQAPGQGLEWMGV IDPSDSYT SYNQGFTGRFVFSVDTSVSTAYLQISSLKAEDTAVYYC ARGGKRAMDY WGQGTTVTVSS | SEQ ID NO: 101 |
| amino acid sequence of light chain variable region | DVVMTQSPDSLAVSLGERATINCKSS QSLVHSNGNTY LHWYQQKPGQPPKLLIY KVS NRFSGVPDRFSGSGSGTDFTLTISSLQAEDVAVYYC SQSTHVPFT FGGGTKLEIK | SEQ ID NO: 102 |
| nucleotide sequence of heavy chain variable region | CAGGTGCAGCTGGTGCAGAGCGGGTCCGAGCTCAAG AAGCCCGGCGCCTCAGTGAAGGTATCGTGCAAGGCT TCCGGTTACACGTTTACCTCTTATTGGATGAACTGGG TCCGCCAGGCCCCCGGACAGGGGTTGGAATGGATGG GCGTCATTGACCCGTCCGACAGTTACACCAGCTACA ACCAGGGCTTCACTGGCCGTTTCGTCTTCTCCGTGGA CACTTCGGTCTCCACCGCTTACCTTCAAATTTCTAGC CTGAAAGCGGAGGACACCGCCGTGTATTACTGTGCA CGGGGGGGGAAGCGCGCCATGGATTATTGGGGCCAG GGCACGACCGTCACCGTGAGCTCT | SEQ ID NO: 103 |
| nucleotide sequence of light chain variable region | GACGTGGTGATGACCCAGTCGCCCGATTCTTTGGCCG TGTCCCTGGGCGAGCGCGCGACCATCAACTGCAAAT CCAGCCAGTCCCTAGTGCACTCAAATGGCAACACGT ACCTGCACTGGTACCAGCAGAAGCCTGGTCAGCCGC CTAAGCTGCTCATCTACAAGGTTTCGAACCGCTTCTC CGGTGTGCCAGACAGGTTTTCTGGTTCCGGCTCCGGA ACCGACTTCACACTGACCATCTCTTCCCTGCAGGCGG AGGATGTAGCCGTGTACTACTGTTCTCAGTCCACCCA TGTGCCCTTTACTTTCGGTGGGGGCACTAAACTGGAG ATCAAG | SEQ ID NO: 104 |
| scFv amino acid sequence | DVVMTQSPDSLAVSLGERATINCKSSQSLVHSNGNTYL HWYQQKPGQPPKLLIYKVSNRFSGVPDRFSGSGSGTDF TLTISSLQAEDVAVYYCSQSTHVPFTFGGGTKLEIKGG GGSGGGGSGGGGSQVQLVQSGSELKKPGASVKVSCK ASGYTFTSYWMNWVRQAPGQGLEWMGVIDPSDSYTS YNQGFTGRFVFSVDTSVSTAYLQISSLKAEDTAVYYCA RGGKRAMDYWGQGTTVTVSS | SEQ ID NO: 105 |
| scFv nucleotide sequence | GACGTGGTGATGACCCAGTCGCCCGATTCTTTGGCCG TGTCCCTGGGCGAGCGCGCGACCATCAACTGCAAAT CCAGCCAGTCCCTAGTGCACTCAAATGGCAACACGT ACCTGCACTGGTACCAGCAGAAGCCTGGTCAGCCGC CTAAGCTGCTCATCTACAAGGTTTCGAACCGCTTCTC CGGTGTGCCAGACAGGTTTTCTGGTTCCGGCTCCGGA ACCGACTTCACACTGACCATCTCTTCCCTGCAGGCGG AGGATGTAGCCGTGTACTACTGTTCTCAGTCCACCCA TGTGCCCTTTACTTTCGGTGGGGGCACTAAACTGGAG ATCAAGGGCGGAGGCGGGAGCGGCGGCGGCGGA AGTGGCGGAGGTGGCAGCCAGGTGCAGCTGGTGCA GAGCGGGTCCGAGCTCAAGAAGCCCGGCGCCTCAGT GAAGGTATCGTGCAAGGCTTCCGGTTACACGTTTACC TCTTATTGGATGAACTGGGTCCGCCAGGCCCCCGGAC AGGGGTTGGAATGGATGGGCGTCATTGACCCGTCCG ACAGTTACACCAGCTACAACCAGGGCTTCACTGGCC GTTTCGTCTTCTCCGTGGACACTTCGGTCTCCACCGC TTACCTTCAAATTTCTAGCCTGAAAGCGGAGGACACC GCCGTGTATTACTGTGCACGGGGGGGGAAGCGCGCC ATGGATTATTGGGGCCAGGGCACGACCGTCACCGTG AGCTCT | SEQ ID NO: 106 |

### Example 4: Confirmation of specificity for CD47 of humanized 3A5 antibody - ELISA and flow cytometer

### 4-1: ELISA analysis

In the present invention, in order to confirm the specificity of 16 humanized antibodies established in Example 3 for CD47, ELISA analysis was performed.

First, in order to encode the CD47 peptide, the CD47 protein (Acrobiosystems, cat#CD7-HA2E9) was dispensed in a 96-well plate at a concentration of 100 ng/well, and then reacted at 4°C overnight. Then, after treatment with 1 X PBST containing 3% BSA, blocking at room temperature for 30 minutes.

The purified 0.8 µg humanized antibody was treated in each well, and then reacted at room temperature for 2 hours, and then washed 3 times with 1 X PBST. Secondary antibody (anti-HRP, 1:5,000) was treated and reacted at room temperature for 30 minutes, washed 3 times with 1 X PBST, and then treated with TMB for color development and reacted at room temperature for 5 minutes. Finally, the reaction was terminated by treatment with a stop solution of 1N H₂SO₄, and then the absorbance was measured at 450 nm.

**[Table 211**

| | ELISA Experimental Conditions | |
|---|---|---|
| ELISA reader | | Infinite F50 |
| Measurement Filter | | 450 nm |
| Measurement Mode | | Single Point Photo |
| Antigen Coating | | 100 ng/well |
| 2nd Antibody (Anti-mIgG-HRP) | | 1:5,000 dilution |
| Substrate | | TMB |

**[Table 22]**

| ELISA test results | |
|---|---|
| Antibody type | OD450 measurements |
| Hu3A5(V1) | 2.5827 |
| Hu3A5(V2) | 2.6904 |
| Hu3A5 (V3) | 2.5426 |
| Hu3A5 (V4) | 2.7364 |
| Hu3A5 (V5) | 2.5703 |
| Hu3A5 (V6) | 2.624 |
| Hu3A5 (V7) | 2.674 |
| Hu3A5 (V8) | 2.4529 |
| Hu3A5 (V9) | 2.647 |
| Hu3A5 (V10) | 2.8076 |
| Hu3A5 (V11) | 2.8133 |
| Hu3A5 (V12) | 2.8549 |
| Hu3A5 (V13) | 0.0966 |
| Hu3A5 (V14) | 0.1467 |
| Hu3A5 (V15) | 0.1114 |
| Hu3A5 (V16) | 0.106 |
| negative control | 0.0431 |

As a result, as shown in Table 22, it was confirmed that Hu3A5 (V1), Hu3A5 (V2), Hu3A5 (V3), Hu3A5 (V4), Hu3A5 (V5), Hu3A5 (V1), Hu3A5 (V6), Hu3A5 (V7), Hu3A5 (V8), Hu3A5 (V9), Hu3A5 (V10), Hu3A5 (V11), Hu3A5 (V12), Hu3A5 (V13), Hu3A5 (V14), Hu3A5 (V15) or Hu3A5 (V16) antibody specifically bound to CD47.

### 4-2: Analysis using flow cytometer

In the present invention, in order to confirm the specificity of 16 humanized 3A5 antibodies established in Example 3 for CD47, flow cytometer analysis was performed.

First, the CD47 overexpressing breast cancer cell line MCF-7 (1 × 10⁷) was reacted with each of 16 humanized 3A5 antibodies (1 µg) for 30 minutes, and then the surface was stained with a secondary antibody, followed by measurement by flow cytometry.

As a positive control, a CD47 antibody (Biolegend PE anti-human CD47, cat# 323108, 5 µl) was used, and as a secondary antibody, a PE-conjugated goat anti-mouse IgG antibody (PE-conjugated goat anti-mouse IgG; Biolegend Inc., cat# 405307, USA, 5 µl) was used.

As a result, as shown in FIG. 2, it was confirmed that all of 16 humanized 3A5 antibodies specifically boun to CD47-overexpressing cells.

### Example 5: Confirmation of blocking ability of CD47-SIRPα binding by humanized 3A5 antibody

Binding of CD47 expressed in cancer cells to SIRPα on macrophages negatively regulates phagocytosis to induce immune evasion in cancer cells. In the present invention, it was attempted to determine whether the humanized Hu3A5 (V10) antibody blocks the CD47-SIRPα interaction.

MCF-7 cells expressing CD47 were treated with Hu3A5(V10) antibody at a concentration of 10⁻¹, 10⁰, 10¹, 10², 10³, 10⁴, 10⁵, 10⁶ and 10⁷ ng/ml, respectively. Hu3A5 (V10) antibody was allowed to bind to MCF-7 cells. Then, after reacting the Hu3A5 (V10) antibody-attached MCF-7 cells with PE-attached SIRPα protein (Acrobiosystems, cat#SIA-HP252), the degree of SIRPα binding to CD47 on the cell surface was measured by flow cytometry.

As a result, as shown in FIG. 3, it was confirmed that the humanized 3A5(V10) antibody of the present invention effectively blocked CD47-SIRPα binding.

### Example 6: Confirmation of effect of enhancing macrophage phagocytosis by humanized 3A5 antibody

Peripheral blood mononuclear cells (PBMCs) from normal human blood were separated by Ficoll-Paque, put in a 24-well plate containing AIM-V media, waited for monocytes to attach to the bottom, and then monocytes were transformed into macrophages. It was cultured for 7 days in AIM-V medium for differentiation.

Peripheral blood mononuclear cells (PBMCs) were co-cultured with Jurkat cells to which CFSE was attached, and then added to the co-culture plate in which the macrophages were differentiated together with Hu3A5 (V10) antibody, and then phagocytosis was analyzed with CFSE⁺ CD14⁺ cells. Hu3A5 (V10) antibody was added at concentrations of 0.01, 0.1, 1, and 10 µg/ml, respectively, and human IgG (hlgG) at a concentration of 10 µg/ml was used as a control.

As a result, as shown in FIG. 4, it was confirmed that the humanized Hu3A5(V10) antibody of the present invention bound to cancer cells overexpressing CD47 and promoted cancer cell phagocytosis of macrophages.

### Example 7: Confirmation of hemagglutination of humanized 3A5 antibody

In the present invention, in order to determine whether the humanized 3A5 antibody induces hemagglutination, it was confirmed whether the Hu3A5 (V10) antibody and the commercial anti-CD47 antibody (clone#CC2C6) were bound to red blood cells/platelets and whether or not to react to hemagglutination.

Blood collected from healthy volunteers was washed three times with PBS containing 1 mmol/f EDTA (ethylenediaminetetraacetic acid), and the washed blood was diluted in PBS containing 1 mmol/ℓ EDTA at a ratio of 1:400 and human RBC (red blood cells) were prepared. Washed RBCs were dispensed at 100 µl/well in 96-well round culture plate, and anti-CD47 antibody (anti-human CD47 mAb, CC2C6 clone) and humanized Hu3A5 (V10) antibody of the present invention were treated at concentrations of 0, 0.1, 0.5, 1, 5, 10, and 25 µg/ml, respectively. The 96-well plate to which the antibody was added was incubated in an incubator at 37°C for 2 hours to confirm RBC hemagglutination.

As shown in Fig. 5, the commercial anti-CD47 antibody (clone#CC2C6) reacted with red blood cells to induce hemagglutination, whereas the humanized Hu3A5(V10) antibody of the present invention did not bind to red blood cells and platelets (Fig. 5A), and did not induce hemagglutination (FIG. 5B).

### Example 8: Confirmation of growth inhibitory efficacy of CD47 overexpressing tumors by 3A5 antibody

In the present invention, in order to confirm whether the 3A5 antibody inhibits the growth of CD47-expressing tumors, an animal experiment was performed as shown in the schematic diagram of FIG. 6A.

First, murine colon adenocarcinoma cells (MC38-hCD47, Biocytogen) expressing 2.5 × 10⁶ human CD47 were subcutaneously implanted with C57BL/6 (6 weeks old, female) mice. On the 10th day after cancer cell transplantation, the size of the cancer tissue of each mouse was measured and uniformly divided into 4 groups as follows (n=5 per group).
1) Rat IgG administration group (control group)
2) Anti-PD-1 antibody administration group
3) Anti-CD47 antibody (3A5 antibody) administration group
4) Anti-PD-1 antibody and anti-CD47 antibody (3A5 antibody) combination administration group

For each experimental group, 200 µg of antibody was administered intraperitoneally to each group, and a total of three doses were administered at intervals of 5 days. The growth of cancer tissue was measured by periodically measuring the size of the cancer tissue together with the administration of the antibody.

As a result, as shown in FIG. 6B, it was confirmed that the growth of tumors overexpressing CD47 was inhibited in 3A5 antibody alone group, and in particular, the tumor growth inhibitory effect of 3A5 antibody and anti-PD-1 antibody combination group was the best. This means that when the anti-PD-1 antibody, which is an immune checkpoint inhibitor, and the 3A5 antibody of the present invention were co-administered, a synergistic effect on tumor treatment was exhibited.

### Example 9: Confirmation of growth inhibitory efficacy of CD47 overexpressing tumors by humanized 3A5 antibody

To confirm the tumor growth inhibitory efficacy of the humanized 3A5 antibody, the mouse CD47 and SIRPα genes were replaced with human CD47 and human SIRPα genes in C57BL/6-hCD47/hSIRPα knock-in mice (C57BL/6-hCD47/hSIRPα knock-in mouse, hCD47 KI) were prepared.

2.5 × 10⁶ human CD47-expressing murine colon adenocarcinoma cells (MC38-hCD47, Biocytogen) were subcutaneously transplanted with 25 of C57BL/6-hCD47/hSIRPα (hCD47 KI, female, 6 weeks old). After measuring the cancer tissue size of each mouse on the 10th day after cancer cell transplantation, 20 of 25 mice were selected and uniformly divided into 4 groups as follows (n=5 per group).
1) Rat IgG administration group (control group)
2) Anti-PD-1 antibody administration group
3) Anti-CD47 antibody (3A5 antibody) administration group
4) Anti-PD-1 antibody and anti-CD47 antibody (3A5 antibody) combination administration group

For each experimental group, 200 µg of the antibody was administered intraperitoneally to each group, and a total of 4 doses were administered at intervals of 5 days. The growth of cancer tissue was measured by periodically measuring the size of the cancer tissue together with the administration of the antibody.

As in Example 10, mouse colon adenocarcinoma cells expressing human CD47 were transplanted into mice, and then a control antibody (Rat IgG), an anti-PD-1 antibody, an anti-CD47 antibody (Hu3A5(V10) antibody), and an anti -PD-1 antibody + anti-CD47 antibody (Hu3A5(V10) antibody) were administered, respectively (FIG. 7A).

As a result, it was confirmed that tumor growth was inhibited in the Hu3A5(V10) antibody alone group, and in particular, it was confirmed that the tumor growth inhibitory effect of the Hu3A5(V10) antibody and anti-PD-1 antibody combination group was the best.

In addition, after the last antibody administration, the main organs (liver, lung, kidney) of the C57BL/6-hCD47/hSIRPα knock-in mouse were isolated on the 32nd day after the end of the experiment with cancer cell transplantation, immersed in 10% formalin solution, and the tissue was fixed for one day. Each fixed tissue was embedded in paraffin wax, and sections were made with a thickness of 5 µm and attached to a glass slide. Each slide was stained with hematoxylin and eosin (H&E) using an H&E staining kit (Hematoxylin and Eosin stain kit; VECTOR laboratories, CAT #: H-3502). Each tissue that has been stained was imaged using a slide scanner (Vectra Polaris Imaging system, PerkinElmer).

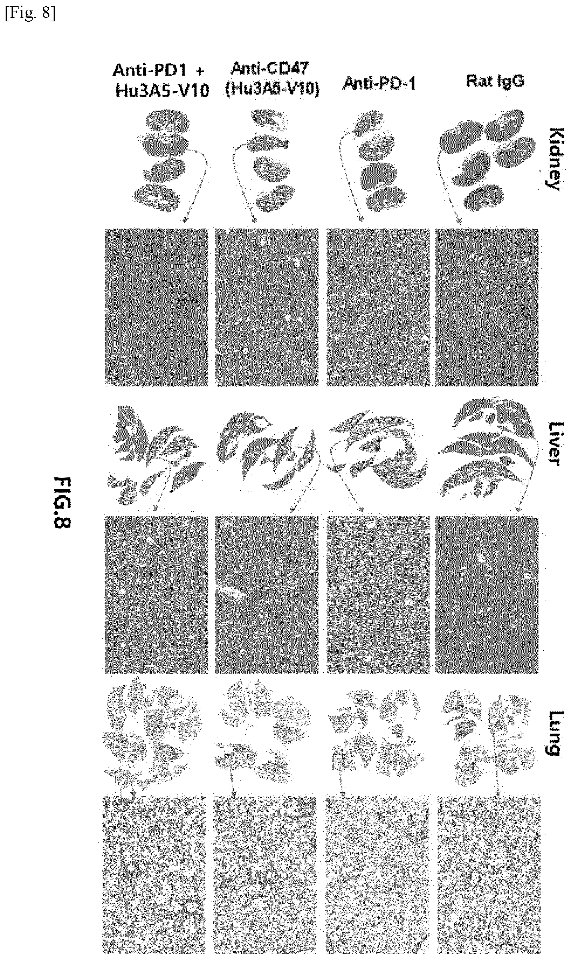

As a result, as shown in FIG. 8, no major tissue damage due to inflammation was observed even after administration of the antibody.

### [Industrial Applicability]

The humanized anti-CD47 antibody of the present invention not only blocks CD47-SIRPα binding, but also promotes phagocytosis by macrophages by binding to CD47-overexpressing cells, and can inhibit the growth of CD47-expressing tumors. Thus, the present humanized anti-CD47 antibody can be applied to the prevention or treatment of diseases or tumors in which the immune response by overexpression of CD47 is suppressed.

## Claims

1. A humanized antibody or fragment thereof that specifically binds to CD47, comprising:
(1) a heavy chain variable region represented by the amino acid sequence of SEQ ID NO: 11 and a light chain variable region represented by the amino acid sequence of SEQ ID NO: 12;
(2) a heavy chain variable region represented by the amino acid sequence of SEQ ID NO: 17 and a light chain variable region represented by the amino acid sequence of SEQ ID NO: 18;
(3) a heavy chain variable region represented by the amino acid sequence of SEQ ID NO: 23 and a light chain variable region represented by the amino acid sequence of SEQ ID NO: 24;
(4) a heavy chain variable region represented by the amino acid sequence of SEQ ID NO: 29 and a light chain variable region represented by the amino acid sequence of SEQ ID NO: 30;
(5) a heavy chain variable region represented by the amino acid sequence of SEQ ID NO: 35 and a light chain variable region represented by the amino acid sequence of SEQ ID NO: 36;
(6) a heavy chain variable region represented by the amino acid sequence of SEQ ID NO: 41 and a light chain variable region represented by the amino acid sequence of SEQ ID NO: 42;
(7) a heavy chain variable region represented by the amino acid sequence of SEQ ID NO: 47 and a light chain variable region represented by the amino acid sequence of SEQ ID NO: 48;
(8) a heavy chain variable region represented by the amino acid sequence of SEQ ID NO: 53 and a light chain variable region represented by the amino acid sequence of SEQ ID NO: 54;
(9) a heavy chain variable region represented by the amino acid sequence of SEQ ID NO: 59 and a light chain variable region represented by the amino acid sequence of SEQ ID NO: 60;
(10) a heavy chain variable region represented by the amino acid sequence of SEQ ID NO: 65 and a light chain variable region represented by the amino acid sequence of SEQ ID NO: 66;
(11) a heavy chain variable region represented by the amino acid sequence of SEQ ID NO: 71 and a light chain variable region represented by the amino acid sequence of SEQ ID NO: 72;
(12) a heavy chain variable region represented by the amino acid sequence of SEQ ID NO: 77 and a light chain variable region represented by the amino acid sequence of SEQ ID NO: 78;
(13) a heavy chain variable region represented by the amino acid sequence of SEQ ID NO: 83 and a light chain variable region represented by the amino acid sequence of SEQ ID NO: 84;
(14) a heavy chain variable region represented by the amino acid sequence of SEQ ID NO: 89 and a light chain variable region represented by the amino acid sequence of SEQ ID NO: 90;
(15) a heavy chain variable region represented by the amino acid sequence of SEQ ID NO: 95 and a light chain variable region represented by the amino acid sequence of SEQ ID NO: 96; or
(16) a heavy chain variable region represented by the amino acid sequence of SEQ ID NO: 101 and a light chain variable region represented by the amino acid sequence of SEQ ID NO: 102.

2. A polynucleotide encoding the humanized antibody or fragment thereof that specifically binds to the CD47 of claim 1.

3. A vector comprising the polynucleotide encoding the humanized antibody or fragment thereof that specifically binds to the CD47 of claim 1.

4. A recombinant cell producing a humanized antibody or fragment thereof that specifically binds to CD47, transformed with the vector of claim 3.

5. A pharmaceutical composition for preventing or treating a disease mediated by cells overexpressing CD47, comprising the humanized antibody or fragment thereof that specifically binds to CD47 of claim 1.

6. The pharmaceutical composition for preventing or treating diseases mediated by cells overexpressing CD47 according to claim 5, wherein the humanized antibody or fragment thereof that specifically binds to CD47 contained in the composition prevents CD47 from interacting with signal-regulating-protein α (SIRPα), or promotes macrophage-mediated phagocytosis against CD47 overexpressing cells.

7. The pharmaceutical composition for preventing or treating a disease mediated by cells overexpressing CD47 according to claim 5, wherein the composition further comprises an immune checkpoint inhibitor.

8. The pharmaceutical composition for preventing or treating a disease mediated by cells overexpressing CD47 according to claim 7, wherein the immune checkpoint inhibitor is an anti-PD-1 antibody.

9. The pharmaceutical composition for preventing or treating a disease mediated by cells overexpressing CD47 according to claim 5, wherein the disease mediated by the CD47 overexpressing cells is a cancer or tumor overexpressing CD47.

10. The pharmaceutical composition for preventing or treating a disease mediated by cells overexpressing CD47 according to claim 9, wherein the cancer or tumor is selected from the group consisting of hematological cancer, ovarian cancer, colon cancer, breast cancer, lung cancer, myeloma, neuroblast-derived CNS tumor, monocytic leukemia, B-cell leukemia, T-cell leukemia, B-cell lymphoma, T-cell lymphoma, and mast cell induced tumor.
